# EUROPEAN PATENT SPECIFICATION

(11) **EP 3 784 349 B1**
(45) Date of publication and mention of the grant of the patent: **27.12.2023**
(21) Application number: 19727500.1
(22) Date of filing: 22.04.2019
(51) Int. Cl.: A61P 31/18, A61K 31/58, C07J 63/00, C12N 9/50, A61K 31/5365, A61K 45/06, A61K 31/4985

(54) **COMPOUNDS WITH HIV MATURATION INHIBITORY ACTIVITY**
VERBINDUNGEN MIT HIV-REIFUNGSHEMMENDER WIRKUNG
COMPOSÉS AYANT UNE ACTIVITÉ INHIBITRICE DE LA MATURATION DU VIH

(30) Priority: 24.04.2018 US 201862661780 P; 05.12.2018 US 201862775527 P
(43) Date of publication of application: 03.03.2021
(73) Proprietor: VIIV Healthcare UK (No.5) Limited, Stevenage SG1 2NY (GB)
(72) Inventor: KADOW, John F., Branford, Connecticut 06405 (US); NAIDU, B. Narasimhulu, Branford, Connecticut 06405 (US)
(74) Representative: Billson, Siân Catherine
(86) International application number: PCT/IB2019/053309
(87) International publication number: WO 2019/207460

(56) References cited:
- WO-A1-2015/157483
- WO-A1-2017/134596
- MENDES E ET AL: "Synthesis, stability and in vitro dermal evaluation of aminocarbonyloxymethyl esters as prodrugs of carboxylic acid agents", BIOORGANIC & MEDICINAL CHEMISTRY : A TETRAHEDRON PUBLICATION FOR THE RAPID DISSEMINATION OF FULL ORIGINAL RESEARCH PAPERS AND CRITICAL REVIEWS ON BIOMOLECULAR CHEMISTRY, MEDICINAL CHEMISTRY AND RELATED DISCIPLINES, ELSEVIER, NL, vol. 10, no. 3, 1 March 2002 (2002-03-01), pages 809-816, XP002296488, ISSN: 0968-0896, DOI: 10.1016/S0968-0896(01)00336-4

## Description

### FIELD OF THE INVENTION

The present invention relates to compounds, pharmaceutical compositions, and use thereof for treating a subject infected with HIV.

### BACKGROUND OF THE INVENTION

Human immunodeficiency virus type 1 (HIV-1) leads to the contraction of acquired immune deficiency disease (AIDS). The number of cases of HIV continues to rise, and currently over twenty-five million individuals worldwide suffer from the virus. Presently, long-term suppression of viral replication with antiretroviral drugs is the only option for treating HIV-1 infection. Indeed, the U.S. Food and Drug Administration has approved twenty-five drugs over six different inhibitor classes, which have been shown to greatly increase patient survival and quality of life. However, additional therapies are still required because of undesirable drug-drug interactions; drug-food interactions; non-adherence to therapy; and drug resistance due to mutation of the enzyme target.

Currently, almost all HIV positive patients are treated with therapeutic regimens of antiretroviral drug combinations termed, highly active antiretroviral therapy ("HAART"). However, HAART therapies are often complex because a combination of different drugs must be administered often daily to the patient to avoid the rapid emergence of drug-resistant HIV-1 variants. Despite the positive impact of HAART on patient survival, drug resistance can still occur. The emergence of multidrug-resistant HIV-1 isolates has serious clinical consequences and must be suppressed with a new drug regimen, known as salvage therapy.

Current guidelines recommend that salvage therapy includes at least two, and preferably three, fully active drugs. Typically, first-line therapies combine three to four drugs targeting the viral enzymes reverse transcriptase and protease. One option for salvage therapy is to administer different combinations of drugs from the same mechanistic class that remain active against the resistant isolates. However, the options for this approach are often limited, as resistant mutations frequently confer broad cross-resistance to different drugs in the same class. Alternative therapeutic strategies have recently become available with the development of fusion, entry, and integrase inhibitors. However, resistance to all three new drug classes has already been reported both in the lab and in patients. Sustained successful treatment of HIV-1-infected patients with antiretroviral drugs will therefore require the continued development of new and improved drugs with new targets and mechanisms of action.

Presently, long-term suppression of viral replication with antiretroviral drugs is the only option for treating HIV-1 infection. To date, a number of approved drugs have been shown to greatly increase patient survival. However, therapeutic regimens known as highly active antiretroviral therapy (HAART) are often complex because a combination of different drugs must be administered to the patient to avoid the rapid emergence of drug-resistant HIV-1 variants. Despite the positive impact of HAART on patient survival, drug resistance can still occur.

The HIV Gag polyprotein precursor (Pr55Gag), which is composed of four protein domains - matrix (MA), capsid (CA), nucleocapsid (NC) and p6 - and two spacer peptides, SP1 and SP2, represents a new therapeutic target. Although the cleavage of the Gag polyprotein plays a central role in the progression of infectious virus particle production, to date, no antiretroviral drug has been approved for this mechanism.

In most cell types, assembly occurs at the plasma membrane, and the MA domain of Gag mediates membrane binding. Assembly is completed by budding of the immature particle from the cell. Concomitant with particle release, the virally encoded PR cleaves Gag into the four mature protein domains, MA, CA, NC and p6, and the two spacer peptides, SP1 and SP2. Gag-Pol is also cleaved by PR, liberating the viral enzymes PR, RT and IN. Gag proteolytic processing induces a morphological rearrangement within the particle, known as maturation. Maturation converts the immature, donut-shaped particle to the mature virion, which contains a condensed conical core composed of a CA shell surrounding the viral RNA genome in a complex with NC and the viral enzymes RT and IN. Maturation prepares the virus for infection of a new cell and is absolutely essential for particle infectivity.

Bevirimat (PA-457) is a maturation inhibitor that inhibits the final step in the processing of Gag, the conversion of capsid-SP1 (p25) to capsid, which is required for the formation of infectious viral particles. Bevirimat has activity against ART-resistant and wild-type HIV and has shown synergy with antiretrovirals from all classes. Bevirimat reduced HIV viral load by a mean of 1.3 log₁₀/mL in patients who achieved trough levels of >= 20 µg/mL and who did not have any of the key baseline Gag polymorphisms at Q369, V370 or T371. However, Bevirimat users with Gag polymorphisms at Q369, V370 or T371 demonstrated significantly lower load reductions than patients without Gag polymorphisms at these sites.

Other examples of maturation inhibitors can be found in PCT Patent Application No. WO2011/100308, PCT Patent Application No. PCT/US2012/024288, Chinese PCT Application No. PCT/CN2011/001302, Chinese PCT Application No. PCT/CN2011/001303, Chinese PCT Application No. PCT/CN2011/002105, PCT/CN2011/002159, WO2013/090664, WO2013/123019, WO 2013/043778, WO 2014/123889, WO 2011/153315, WO 2011/153319, WO 2012/106188, WO 2012/106190, WO 2013/169578, WO 2014/13081.

The following three compounds are inhibitors of HIV maturation and as described in previously published patent applications, have utility for the treatment of HIV. They will be referred to as Parent Maturation Inhibitors 1, 2, and 3 as shown in the scheme below. They may be abbreviated as Ml 1, Ml 2, and MI 3.

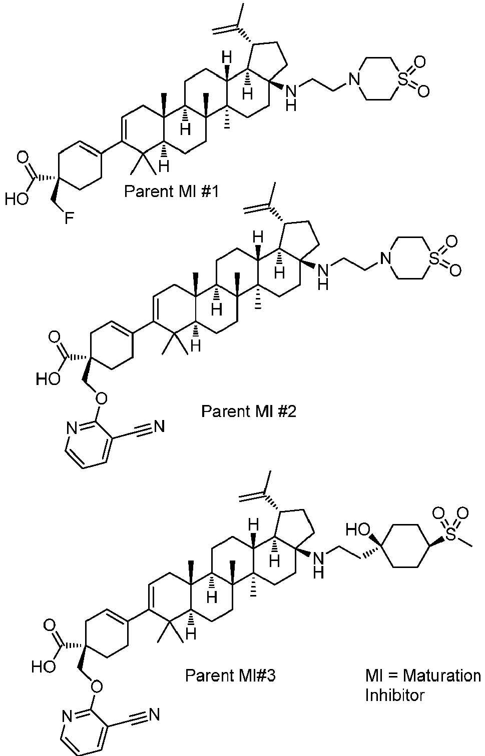

MI 1 can be prepared, for example, as described in WO2015/157483. MI 2 and MI 3 can be prepared, for example, as described in WO2017/134596.

### SUMMARY OF THE INVENTION

Briefly, in one aspect, the present invention provides compounds of Formula I or a pharmaceutically acceptable salt thereof 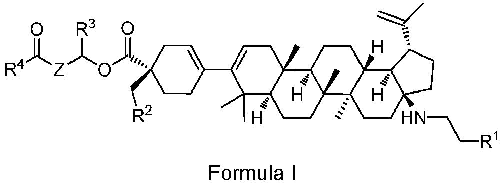
wherein R¹ is 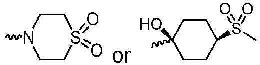 where the squiggly line indicates the point of attachment to the rest of the molecule;
R² is For 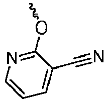 where the squiggly line indicates the point of attachment to the rest of the molecule;
R³ is H or CH₃;
Z is O and
R⁴ is C₁₋₃₀ alkyl.

In another aspect, the present invention provides pharmaceutical compositions comprising a compound or salt of the invention.

In another aspect, the present invention provides a compound or salt of the invention for use in therapy.

In another aspect, the present invention provides a compound of the invention for use in treating an HIV infection.

### BRIEF DESCRIPTION OF THE FIGURES

Figure 1 is a graph of concentration vs. time from plasma of MI#3 in ng / mL vs time in hours following subcutaneous (SQ) doses of MI3# or the prodrugs from Examples 3, 4, and 5 as suspensions
Figure 2 is a concentration vs time curve of the plasma concentrations of parent MI #1 following a single Intramuscular (IM) administration of the Prodrugs and parent Ml #1 to male SD rats.

### DETAILED DESCRIPTION OF REPRESENTATIVE EMBODIMENTS

The compounds and salts of this invention are designed to provide enhanced efficacy or utility for the treatment of HIV by either enhancing the exposure of the parent compound after oral dosing or by extending the dosing intervals (increasing time needed between doses or said another way, reducing the frequency of doses) that are required compared to treatment with the parent molecules. In addition, in some cases the prodrugs may provide reduced side effects and / or improved tolerability due to changes in the rate of free drug reaching systemic exposure or changes in the method or route of administration.

Preferably, the subject to be treated is a human.

Pharmaceutically acceptable salts may be derived from a variety of organic and inorganic counter ions well known in the art and include, by way of example only, sodium, potassium, calcium, magnesium, ammonium, and tetraalkylammonium, and when the molecule contains a basic functionality, salts of organic or inorganic acids, such as hydrochloride, hydrobromide, tartrate, mesylate, acetate, maleate, lysine, and oxalate. Suitable salts include those described in P. Heinrich Stahl, Camille G. Wermuth (Eds.), Handbook of Pharmaceutical Salts Properties, Selection, and Use; 2002.

Preferred compounds of this invention include the following (only those with Z = O and R4 = C1-30 alkyl are compounds of the invention; any others are considered as comparative examples)

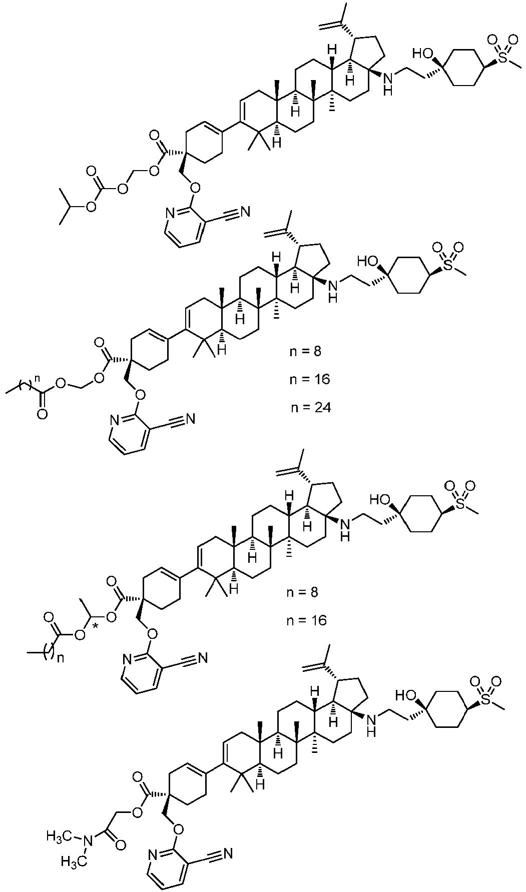

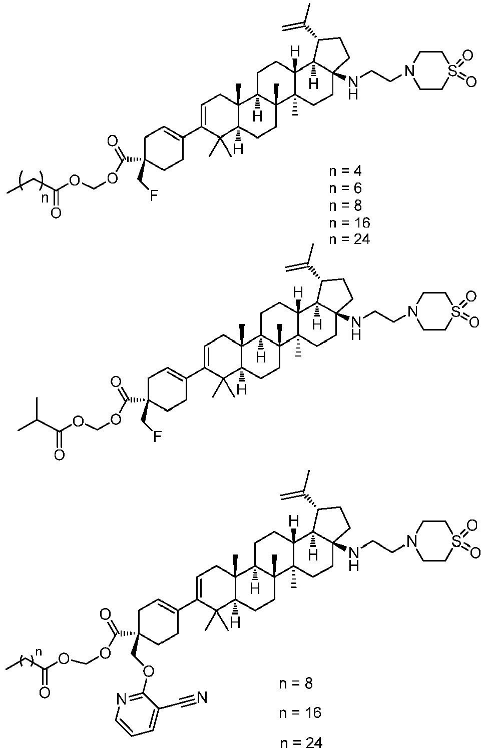

For medical uses, preferred routes of administration are oral and by injection to deliver subcutaneously. Therefore, preferred pharmaceutical formulations of this invention include tablets, capsules, and forms suitable for injection.

The present invention is also directed to a compound of the present invention for use in treating an HIV infection further comprising administration of at least one other agent used for treatment of AIDS or HIV infection selected from the group consisting of nucleoside HIV reverse transcriptase inhibitors, non-nucleoside HIV reverse transcriptase inhibitors, HIV protease inhibitors, HIV fusion inhibitors, HIV attachment inhibitors, CCR5 inhibitors, CXCR4 inhibitors, HIV budding or maturation inhibitors, and HIV integrase inhibitors. Preferred additional agents include, for example, FTC, ibalizumab, PRO-140, dolutegravir, abacavir lamivudine, fasamprenavir, rilpivirine, atazanavir, darunavir, MK-8718, MK-8591, tenofovir alfenamide, cabotegravir, and bictegravir. Particular preferred additional agents are dolutegravir, and cabotegravir.

The present invention may be used in combination with one or more agents useful as pharmacological enhancers as well as with or without additional compounds for the prevention or treatment of HIV. Examples of such pharmacological enhancers (or pharmacokinetic boosters) include ritonavir and Cobicistat (formerly GS-9350).

### EXAMPLES

### Chloromethyl hexacosanoate

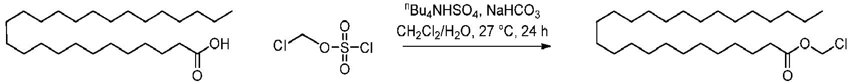

To a stirred solution of hexacosanoic acid (300 mg, 0.756 mmol) in DCM (10 mL) and water (10 mL) was added sodium bicarbonate (254 mg, 3.03 mmol) and tetrabutylammonium hydrogen sulfate (25.7 mg, 0.076 mmol) followed by chloromethyl sulfochloridate (150 mg, 0.908 mmol) drop wise at 0 °C. The reaction mixture was allowed to warm to 27 °C and stirred for 24 hrs. The progress of the reaction was monitored by TLC (SiO₂, 10% EtOAc/ Pet., Rf = 0.8, Iodine-active). The reaction mixture was diluted with water (10 mL) and extracted with DCM (2 × 20 mL). The combined organic phases were washed with brine (2 × 50 mL), dried over Na₂SO₄, filtered and concentrated under reduced pressure to afford chloromethyl hexacosanoate (150 mg, Yield: 42%, pale yellow solid). ¹H NMR (400MHz, CDCl₃) δ = 5.70 (s, 2H), 2.43-2.31 (m, 2H), 1.65 (quin, *J* = 7.5 Hz, 2H), 1.39-1.17 (m, 44H), 0.88 (t, *J* = 7.9 Hz, 3H). GCMS: RT = 13.72 mins, M = 444.5.

### 1-Chloroethyl stearate

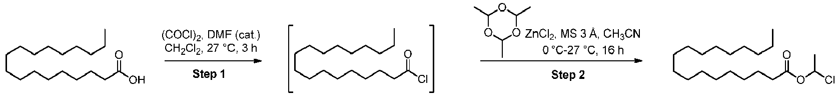

Step 1: To a stirred solution of stearic acid (2 g, 7.03 mmol) in DCM (20 mL) and DMF (0.100 mL) was added oxalyl chloride (0.800 mL, 9.14 mmol) at 0 °C. Then, the reaction mixture was allowed to warm to 27 °C and stirred for 3 hr. The progress of the reaction was monitored by TLC (small aliquant was treated with methanol and analyzed by TLC). It was then concentrated under vacuum to afforded yellow liquid.

Step 2: The above formed crude compound was dissolved in acetonitrile (20 mL) and cooled to 0 °C, molecular sieves (500 mg) followed by zinc chloride (0.192 g, 1.406 mmol) and paraldehyde (0.929 g, 7.03 mmol) were added at 0 °C. The reaction mixture was allowed to warm to 27 °C and stirred for 16 hr. The progress of the reaction was monitored by TLC (SiO₂, 30% EtOAc/pet., Rf = 0.4, KMnO₄-active). On completion of the starting material, the reaction mixture was filtered through a small pad of *Celite* and washed with EtOAc (50 mL). The eluent was concentrated under reduced pressure and finally dried under vacuum to give crude compound as pale yellow oil. The crude compound was purified by normal phase column chromatography (SiO₂, 100-200 mesh, 0-5% EtOAc/pet.). The fractions containing product were collected and concentrated under reduced pressure to afford 1-chloroethyl stearate (140 mg, Yield: 5%, off white solid). ¹H NMR (400MHz, CDCl₃) δ = 6.62-6.50 (m, 1H), 2.39-2.27 (m, 2H), 1.78 (d, *J* = 6.0 Hz, 3H), 1.71-1.58 (m, 2H), 1.39-1.18 (m, 28H), 0.96-0.81 (m, 3H).

### Chloromethyl decanoate

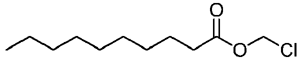

To a stirred solution of decanoic acid (3 g, 17.42 mmol) in DCM (30 mL) and water (30 mL) was added sodium bicarbonate (5.85 g, 69.7 mmol) and tetrabutylammonium hydrogen sulfate (0.591 g, 1.742 mmol) followed by chloromethyl sulfochloridate (3.45 g, 20.90 mmol) drop-wise at 0 °C. Then, the reaction mixture was allowed to warm to 27 °C and stirred for 4 hr. The progress of the reaction was monitored by TLC (SiO₂, 10% EtOAc/Pet. Rf = 0.8, Iodine-active). On completion, the reaction mixture was partitioned between DCM (60 ml) and water (50 mL), separated the organic layer, dried over anhydrous Na₂SO₄, filtered and concentrated under vacuum to afford chloromethyl decanoate (3.8 g, Yield: 89%, pale yellow liquid). ¹H NMR (400MHz, CDCl₃) δ = 5.70 (s, 2H), 2.38 (t, *J* = 7.9 Hz, 2H), 1.69-1.63 (m, 2H), 1.40-1.18 (m, 12H), 0.88 (t, *J* = 8.0 Hz, 3H).

### Chloromethyl stearate

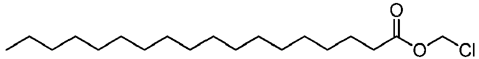

To a stirred solution of stearic acid (3 g, 10.55 mmol) in DCM (30 mL) and water (30 mL) was added sodium bicarbonate (3.54 g, 42.2 mmol) and tetrabutylammonium hydrogen sulfate (0.358 g, 1.055 mmol) followed by chloromethyl sulfochloridate (2.088 g, 12.65 mmol) drop-wise at 0 °C. The reaction mixture allowed to warm to 27 °C and stirred for 4 hr. The progress of the reaction was monitored by TLC (SiO₂, 10% EtOAc/Pet. Rf = 0.8, Iodine-active). The reaction mixture was diluted with water (50 mL) and extracted with DCM (2 × 40 mL). The combined organic phases were washed with brine (2 × 50 mL), dried over anhydrous Na₂SO₄, filtered and concentrated under reduced pressure to afford chloromethyl stearate (3.5 g, Yield: 90%, as off-white solid). ¹H NMR (400MHz, CDCl₃) δ = 5.70 (s, 2H), 2.38 (t, *J* = 7.6 Hz, 2H), 1.69-1.55 (m, 2H), 1.40-1.13 (m, 28H), 0.88 (t, *J* = 6.8 Hz, 3H).

### 1-Chloroethyl decanoate

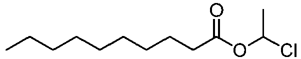

To a solution of decanoic acid (3 g, 17.42 mmol) in DCM (60 mL) stirred under nitrogen at 0 °C was added cat. DMF and neat oxalyl chloride (1.982 mL, 22.64 mmol) drop wise over a period 5 min. The reaction mixture was allowed to warm to 27 °C and stirred for 4 hr. Progress of the reaction was monitored by TLC (small amount of reaction mixture was quenched with methanol and analyzed by TLC, 20% EtOAc/pet. ether, Rf = 0.7, KMnO₄-active). On completion of starting material, the reaction mixture was concentrated under nitrogen atmosphere and co-distilled with toluene (2 × 50 mL) to get decanoyl chloride (3.3 g, Yield: 99%, pale yellow oil). The crude product was used in the next step without any further purification.

To a solution of 2,4,6-trimethyl-1,3,5-trioxane (2.287 g, 17.30 mmol), molecular sieves 4 A° (3 g, 17.30 mmol), and zinc chloride (0.047 g, 0.346 mmol) in acetonitrile (20 mL), decanoyl chloride (3.3 g, 17.30 mmol) in acetonitrile (20 mL) was added at -10 °C stirred under nitrogen atmosphere 1 hr. Then, the reaction mixture was allowed to warm to 27 °C and stirred for 18 hr. Progress of the reaction was monitored by TLC (SiO₂, 5% EtOAc/Pet., Rf = 0.3, KMnO₄-active). On completion, the reaction mixture was diluted with EtOAc (100 mL), filtered through *Celite* pad. The filtrate was concentrated under reduced pressure to give crude residue. The crude residue was purified by column chromatography on silica gel (100-200 mesh, 0-3% EtOAc/Pet.). The fractions containing product were collected and concentrated under reduced pressure to get the desired product along with impurities (600 mg). The above residue was again purified by column chromatography on silica gel (100-200 mesh, 0-3% EtOAc/Pet.). The fractions containing pure product were collected and concentrated to afford 1-chloroethyl decanoate (220 mg, Yield: 5%, pale yellow oil). ¹H NMR (400MHz, CDCl₃) δ = 6.60-6.50 (m, 1H), 2.35 (dt, *J* = 1.0, 7.5 Hz, 2H), 1.78 (d, *J* = 6.0 Hz, 3H), 1.71-1.59 (m, 2H), 1.40-1.20 (m, 12H), 0.92-0.84 (m, 3H).

### 2-Bromo-N,N-dimethylacetamide

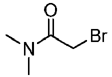

To a stirred solution of 2-bromoacetyl bromide (2 g, 9.91 mmol) and dimethylamine hydrochloride (0.541 g, 6.64 mmol) in DCM (20 mL) was added TEA (2.348 mL, 16.84 mmol) drop wise at -78 °C and stirred the reaction mixture at same temperature for 1 h. Progress of the reaction was monitored by TLC (SiO₂, 50% EtOAc/Pet. Rf = 0.8, KMnO₄-active). The reaction mixture was allowed to warm to 27 °C and diluted with DCM (50 mL) and successively washed with water (2 × 50 mL), 10% aqueous citric acid (100 mL) and then saturated NaHCO₃ (100 mL). The combined organic layer wee dried over Na₂SO₄, filtered and concentrated under reduced pressure to get the crude compound as brown oil. The crude compound was purified by normal phase column chromatography on silica gel (SiO₂, 100-200 mesh, 0-20% EtOAc/Pet.). The fractions containing product were collected and concentrated under reduced pressure to afford 2-bromo-N,N-dimethylacetamide (600 mg, Yield: 30%, pale brown liquid). ¹H NMR (400MHz, CDCl₃) δ = 3.87 (s, 2H), 3.11 (s, 3H), 2.99 (s, 3H). LCMS: RT = 0.99 mins, (M+H) = 166.14, LCMS Purity = 83%.

### Chloromethyl octanoate

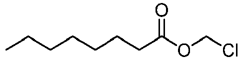

To a stirred solution of octanoic acid (4 g, 27.7 mmol) in DCM (30 mL) and water (30 mL) was added sodium bicarbonate (9.32 g, 111 mmol) and tetrabutylammonium hydrogen sulfate (0.942 g, 2.77 mmol) followed by chloromethyl sulfurochloridate (5.49 g, 33.3 mmol) at 0 °C. Then, the reaction mixture was allowed to warm to 27 °C and stirred for 16 hr. The progress of the reaction was monitored by TLC (SiO₂, 5% EtOAc/Pet. Rf = 0.4, KMnO₄-active). The reaction mixture was quenched with water (100 mL) and extracted with DCM (2 × 100 mL). The combined organic phases were washed with brine (2 × 50 mL), dried over Na₂SO₄, filtered and concentrated under reduced pressure to give the crude residue as pale yellow liquid. The crude compound was purified by normal phase column chromatography on silica gel (SiO₂, 100-200 mesh) with 0-5% EtOAc/Pet. The fractions containing product were collected and concentrated under reduced pressure to afford chloromethyl octanoate (N68486-19-A2, 4.25 g, Yield: 79%, colourless oil). ¹H NMR (400MHz, CDCl₃) δ = 5.70 (s, 2H), 2.38 (t, *J* = 7.6 Hz, 2H), 1.69-1.62 (m, 2H), 1.37-1.24 (m, 8H), 0.88 (t, *J* = 7.2 Hz, 3H). GCMS: RT = 3.72 mins, M = 192.1.

### Chloromethyl dodecanoate

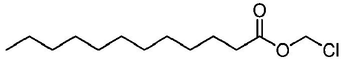

To a stirred solution of dodecanoic acid (2 g, 9.98 mmol) in DCM (20 mL) and water (20 mL) was added sodium bicarbonate (3.35 g, 39.9 mmol) and tetrabutylammonium hydrogen sulfate (0.339 g, 0.998 mmol) followed by chloromethyl sulfurochloridate (1.977 g, 11.98 mmol) at 0 °C. Then, the reaction mixture was allowed to warm to 27 °C and stirred for 3 hr. The progress of the reaction was monitored by TLC (SiO₂, 10% EtOAc/Pet. Rf = 0.6, KMnO₄-active). After 3 hr, the reaction mixture was diluted with DCM (200 mL), washed with water (2 × 50 mL) and brine solution (20 mL). The organic layer was dried over anhydrous Na₂SO₄, filtered and concentrated under reduced pressure to get crude residue. The crude compound was purified by normal phase column chromatography on silica gel (SiO₂, 100-200 mesh) with 0-5% EtOAc/Pet. The fractions containing product were collected and concentrated under reduced pressure to afford chloromethyl dodecanoate (N67709-82-A2, 2 g, Yield: 81%, colourless liquid). ¹H NMR (400MHz, CDCl₃) δ = 5.70 (s, 2H), 2.38 (t, *J* = 7.5 Hz, 2H), 1.69-1.62 (m, 2H), 1.36-1.24 (m, 16H), 0.88 (t, *J* = 7.2 Hz, 3H).

### Chloromethyl acetate

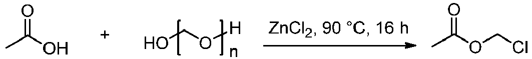

To a mixture of paraformaldehyde (1.913 g, 63.7 mmol) and zinc chloride (0.174 g, 1.274 mmol) was added acetyl chloride (5 g, 63.7 mmol) dropwise at 0 °C. The reaction mixture was allowed to warm to 27 °C and stirred at 90 °C for 16 hr. Then, the reaction mixture was quenched with saturated NaHCO₃ (20 mL) and extracted with Et₂O (2 × 50 mL). The combined organic layers were washed with brine solution (50 mL), dried over Na₂SO₄, filtered and concentrated under reduced pressure to afford the crude residue (4.2 g). The crude compound was purified by fractional distillation at 120 °C to afford pure chloromethyl acetate (1.5 g, Yield: 45%, colourless liquid). ¹H NMR (400 MHz, CDCl₃) δ = 5.69 (s, 2H), 2.15 (s, 3H).

### Chloromethyl butyrate

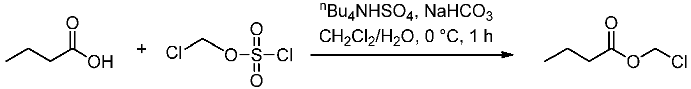

To a stirred solution of butyric acid (2 g, 22.70 mmol) in DCM (30 mL) and water (30.0 mL) was added sodium bicarbonate (7.63 g, 91 mmol) and tetrabutylammonium hydrogen sulfate (0.771 g, 2.270 mmol) followed by chloromethyl sulfurochloridate (5.62 g, 34.0 mmol) drop wise at 0 °C. The reaction mixture was stirred at 0 °C for 1 h. The progress of the reaction was monitored by TLC (SiO₂, 5% EtOAc/Pet., Rf = 0.8, KMnO₄-active). The reaction mixture was quenched with water (20 mL) and extracted with DCM (2 × 25 mL). The combined organic layers were washed with brine (2 × 50 mL) and concentrated under reduced pressure to give the crude residue. The crude compound was dissolved in Et₂O (50 mL) and washed with cold water (4 × 30 mL), dried over Na₂SO₄, filtered and concentrated under reduced pressure to afford chloromethyl butyrate (1.0 g, Yield: 31%, pale yellow liquid).. ¹H NMR (400 MHz, CDCl₃) δ = 5.70 (s, 2H), 2.37 (t, *J* = 7.5 Hz, 2H), 1.74-1.65 (m, 2H), 0.98 (t, *J* = 7.4 Hz, 3H). GCMS: RT = 1.646 mins, Purity = 98%.

### Chloromethyl hexanoate

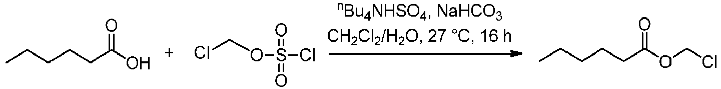

To a stirred solution of hexanoic acid (2 g, 17.22 mmol) in DCM (20 mL) and water (20.00 mL) was added sodium bicarbonate (5.79 g, 68.9 mmol) and tetrabutylammonium hydrogen sulfate (0.585 g, 1.722 mmol) followed by chloromethyl sulfurochloridate (4.26 g, 25.8 mmol) at 0 °C. Then, the reaction mixture was allowed to warm to 27 °C and stirred for 16 hr. The progress of the reaction was monitored by TLC (SiO₂, 5% EtOAC/Pet., Rf = 0.4, KMnO₄-active). The reaction mixture was diluted with water (100 mL) and DCM (200 mL). The aqueous layer was extracted with DCM (2 × 100 mL) and the combined organic layers were washed with brine (2 × 50 mL), dried over Na₂SO₄, filtered and concentrated under reduced pressure to give the crude residue as pale-yellow oil. The crude compound was purified by normal phase column chromatography on silica gel (SiO₂, 100-200 mesh) with 0-5% EtOAc/Pet. The fractions containing product were collected and concentrated under reduced pressure to afford chloromethyl hexanoate (1.8 g, Yield: 49%, colourless oil). ¹H NMR (400 MHz, CDCl₃) δ = 5.70 (s, 2H), 2.38 (t, *J* = 7.5 Hz, 2H), 1.68-1.64 (m, 2H), 1.35-1.29 (m, 4H), 0.89 (t, *J* = 7.2 Hz, 3H). GCMS: RT = 2.336 mins, [M] = 165.0, Purity = 78%.

### Chloromethyl isobutyrate

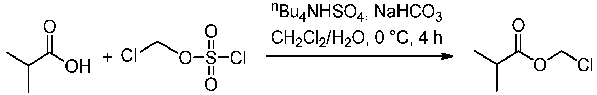

To stirred a solution of isobutyric acid (2 g, 22.70 mmol) in DCM (30 mL) and water (30 mL) was added sodium bicarbonate (7.63 g, 91 mmol) and tetrabutylammonium hydrogen sulfate (0.771 g, 2.270 mmol) followed by chloromethyl sulfurochloridate (5.62 g, 34.0 mmol) drop wise at 0 °C. The reaction mixture was stirred at 0 °C for 4 h. The progress of the reaction was monitored by TLC (SiO₂, 5% EtOAc/Pet., Rf = 0.8, KMnO₄-active). The reaction mixture was quenched with water (20 mL) and extracted with DCM (2 × 25 mL). The combined organic layers were washed with brine (2 × 50 mL) and concentrated under reduced pressure to give the crude residue. The crude compound was redissolved in diethyl ether (50 mL) and washed with cold water (4 × 30 mL), dried over Na₂SO₄, filtered and concentrated under reduced pressure to afford chloromethyl isobutyrate (1 g, Yield: 25%, pale yellow liquid). ¹H NMR (400 MHz, CDCl₃) δ = 5.71 (s, 2H), 2.65-2.58 (m, 1H), 1.23-1.19 (m, 6H).

### Chloromethyl hexanoate

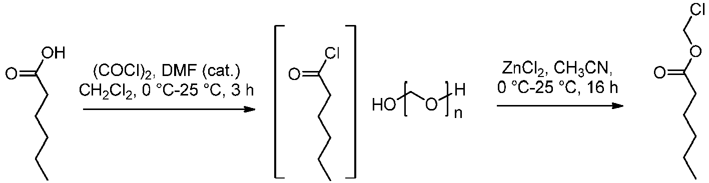

To a stirred solution of hexanoic acid (2 g, 17.22 mmol) in DCM (10 mL) and DMF (0.50 mL) was added oxalyl chloride (1.959 mL, 22.38 mmol) at 0 °C. Then, the reaction mixture was allowed to warm to 27 °C and stirred for 3 hr. The progress of the reaction was monitored by TLC (small aliquant was treated with methanol and analyzed by TLC). It was then concentrated under vacuum to afford yellow liquid which was then dissolved in CH₃CN (10 mL) and cooled to 0 °C. It was then added molecular sieves (500 mg) followed by zinc chloride (0.469 g, 3.44 mmol) and paraformaldehyde (1.034 g, 34.4 mmol) at 0 °C. The reaction mixture was allowed to warm to 27 °C and stirred for 16 hr. The progress of the reaction was monitored by TLC (SiO₂, 5% EtOAc/Pet. Rf = 0.6, KMnO₄-active). On completion, the reaction mixture was filtered through a small pad of *elite* and washed with Et₂O (100 mL). The organic layer was concentrated under reduced pressure at 37 °C and dried under vacuum to afforded crude residue as pale-yellow oil. The crude compound was purified by normal phase column chromatography on silica gel (SiO₂, 100-200 mesh, 0-3% Et₂O/Pet.). The fractions containing product were collected and concentrated under reduced pressure to afford chloromethyl hexanoate (350 mg, Yield: 9%, colourless oil). *Note:* The product is volatile and some of the material was lost during concentration. ¹H NMR (400 MHz, CDCl₃) δ = 5.70 (s, 2H), 2.38 (t, *J* = 7.5 Hz, 2H), 1.68-1.64 (m, 2H), 1.35-1.29 (m, 4H), 0.89 (t, *J* = 7.2 Hz, 3H). GCMS: RT = 2.35 mins, [M] = 165.0.

### Example 1: (S)-((Isopropoxycarbonyl)oxy)methyl 1-(((3-cyanopyridin-2-yl)oxy)methyl)-4-((1R,3aS,5aR,5bR,7aR,11aS,11bR,13aR,13bR)-3a-((2-((1s,4R)-1-hydroxy-4-(methylsulfonyl)cyclohexyl)ethyl)amino)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)cyclohex-3-enecarboxylate

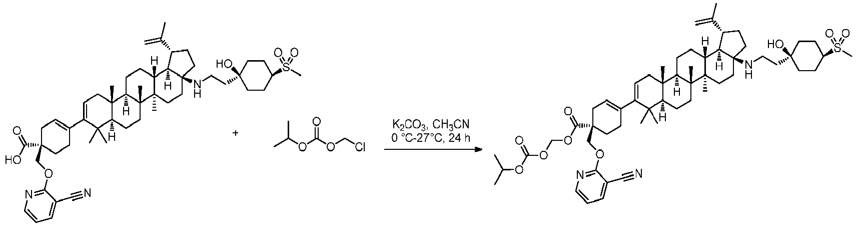

To a stirred solution of (*S*)-1-(((3-cyanopyridin-2-yl)oxy)methyl)-4-((1R,3aS,5aR,5bR,7aR,11aS,11bR,13aR,13bR)-3a-((2-((1s,4R)-1-hydroxy-4-(methylsulfonyl)cyclohexyl)ethyl)amino)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)cyclohex-3-enecarboxylic acid (100 mg, 0.115 mmol) in CH₃CN (15 mL) was added potassium carbonate (23.82 mg, 0.172 mmol) and chloromethyl isopropyl carbonate (21.04 mg, 0.138 mmol) at 0 °C. The reaction mixture was allowed to warm to 27 °C and stirred for 24 hr. Progress of the reaction was monitored by TLC (SiO₂, 5% MeOH/DCM, Rf = 0.3, UV-active, I₂-active). The reaction mixture was diluted with DCM (50 mL) and water (30 mL). Then, the aqueous layer was separated and extracted with DCM (2 × 50 ml). The combined organic layers were washed with brine (30 mL), dried over anhydrous Na₂SO₄ and filtered. The filtrate was concentrated under reduced pressure to afford the crude product. The crude product was purified by normal phase column chromatography on neutral alumina (0-2% MeOH/DCM). The fractions containing product were collected and concentrated under reduced pressure to afford (S)-((isopropoxycarbonyl)oxy)methyl 1-(((3-cyanopyridin-2-yl)oxy)methyl)-4-((1R,3aS,5aR,5bR,7aR,11aS,11bR,13aR,13bR)-3a-((2-((1s,4R)-1-hydroxy-4-(methylsulfonyl)cyclohexyl)ethyl)amino)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)cyclohex-3-enecarboxylate (33 mg, Yield: 28%, off-white solid). ¹H NMR (400MHz, CDCl₃) δ = 8.38-8.27 (m, 1H), 7.87-7.85 (m, 1H), 6.97 (dd, *J* = 5.0, 7.2 Hz, 1H), 5.78 (dd, *J* = 5.6, 19.6 Hz, 2H), 5.37-5.33 (m, 1H), 5.19 (d, *J* = 5.5 Hz, 1H), 4.90-4.84 (m, 1H), 4.74-4.71 (m, 1H), 4.61-4.55 (m, 3H), 2.87-2.64 (m, 7H), 2.56-2.49 (m, 1H), 2.34-1.77 (m, 15H), 1.76-1.58 (m, 8H), 1.49-1.16 (m, 22H), 1.14-0.99 (m, 6H), 0.96 (s, 6H), 0.91 (s, 3H), 0.84 (s, 3H). LCMS (ELSD): RT = 7.24 mins, (M+H) = 986.5. HPLC purity = 99%.

### Example 2: (S)-(Decanoyloxy)methyl 1-(((3-cyanopyridin-2-yl)oxy)methyl)-4-((1R,3aS,5aR,5bR,7aR,11aS,11bR,13aR,13bR)-3a-((2-((1s,4R)-1-hydroxy-4-(methylsulfonyl)cyclohexyl)ethyl)amino)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)cyclohex-3-enecarboxylate

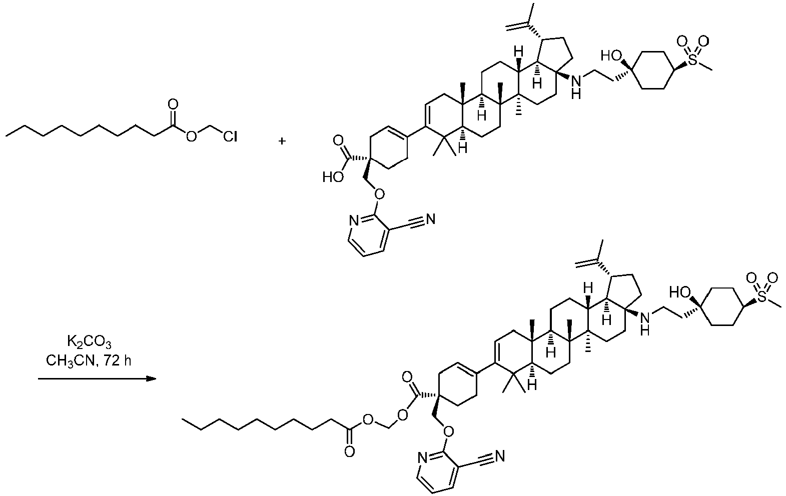

To a stirred solution of (S)-1-(((3-cyanopyridin-2-yl)oxy)methyl)-4-((1R,3aS,5aR,5bR,7aR,11aS,11bR,13aR,13bR)-3a-((2-((1s,4R)-1-hydroxy-4-(methylsulfonyl)cyclohexyl)ethyl)amino)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)cyclohex-3-enecarboxylic acid (300 mg, 0.345 mmol) and chloromethyl decanoate (114 mg, 0.517 mmol) in CH₃CN (10 mL) was added potassium carbonate (191 mg, 1.379 mmol) at 0 °C. Reaction mixture was allowed to warm to 27 °C and stirred for 72 h. The progress of the reaction was monitored by TLC (SiO₂, 10% MeOH/DCM, Rf = 0.6, KMnO₄-active). The reaction mixture was quenched with water (30 mL) and extracted with DCM (2 × 20 mL). The combined organic layers were washed with brine (40 mL), dried over Na₂SO₄, filtered and concentrated under reduced pressure to afford the crude product. The crude product was purified by normal phase column chromatography on neutral alumina with 0-1% MeOH/DCM. The fractions containing product were collected and concentrated under reduced pressure to afford (*S*)-(decanoyloxy)methyl 1-(((3-cyanopyridin-2-yl)oxy)methyl)-4-((1R,3aS,5aR,5bR,7aR,11aS,11bR,13aR,13bR)-3a-((2-((1s,4R)-1-hydroxy-4-(methylsulfonyl)cyclohexyl)ethyl)amino)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)cyclohex-3-enecarboxylate (218 mg, Yield: 59%, white solid). ¹H NMR (400MHz, CDCl₃) δ = 8.32 (dd, *J* = 1.9, 4.9 Hz, 1H), 7.86 (dd, *J* = 1.9, 7.6 Hz, 1H), 6.98 (dd, *J* = 5.0, 7.5 Hz, 1H), 6.39 (br s, 1H), 5.84-5.72 (m, 2H), 5.38-5.30 (m, 1H), 5.21-5.16 (m, 1H), 4.74-4.71 (m, 1H), 4.61-4.58 (m, 1H), 4.57-4.49 (m, 2H), 2.87-2.63 (m, 7H), 2.56-2.49 (m, 1H), 2.30 (t, *J* = 7.6 Hz, 2H), 2.26-1.78 (m, 15H), 1.77-1.55 (m, 11H), 1.53-1.17 (m, 24H), 1.14-1.00 (m, 7H), 0.96 (s, 6H), 0.93-0.81 (m, 10H). LCMS: RT = 4.382 mins, (M+H) = 1054.7. HPLC Purity = 97%.

### Example 3: (S)-(Stearoyloxy)methyl 1-(((3-cyanopyridin-2-yl)oxy)methyl)-4-((1R,3aS,5aR,5bR,7aR,11aS,11bR,13aR,13bR)-3a-((2-((1s,4R)-1-hydroxy-4-(methylsulfonyl)cyclohexyl)ethyl)amino)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)cyclohex-3-enecarboxylate

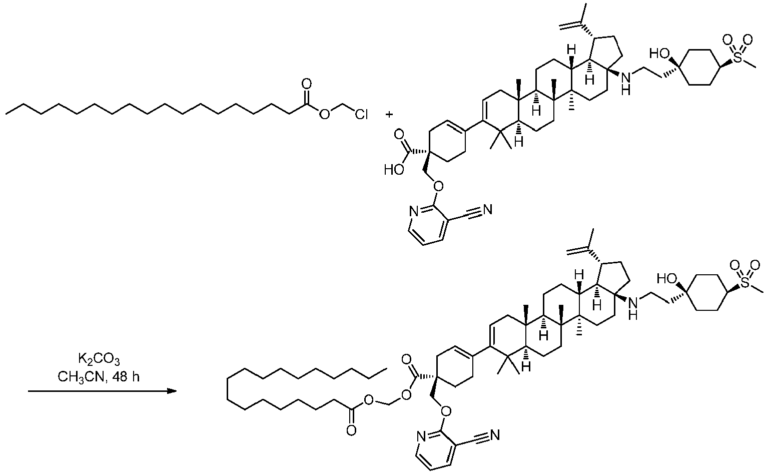

To a stirred solution of (*S*)-1-(((3-cyanopyridin-2-yl)oxy)methyl)-4-((1R,3aS,5aR,5bR,7aR,11aS,11bR,13aR,13bR)-3a-((2-((1s,4R)-1-hydroxy-4-(methylsulfonyl)cyclohexyl)ethyl)amino)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)cyclohex-3-enecarboxylic acid (100 mg, 0.115 mmol) and chloromethyl stearate (77 mg, 0.230 mmol) in acetonitrile (5 mL) was added potassium carbonate (79 mg, 0.575 mmol) at 0 °C. The reaction mixture was allowed to warm to 27 °C and stirred for 48 h. The progress of the reaction was monitored by TLC (SiO₂, 10% MeOH/DCM, Rf = 0.6, KMnO₄-active). The reaction mixture was quenched with water (10 mL) and extracted with DCM (2 × 20 mL). The combined organic layers were washed with brine (20 mL), dried over Na₂SO₄, filtered and concentrated under reduced pressure to afford the crude product. The crude product was purified by normal phase column chromatography on neutral alumina with 0-1% MeOH/DCM. The fractions containing product were collected and concentrated under reduced pressure to afford (*S*)-(stearoyloxy)methyl 1-(((3-cyanopyridin-2-yl)oxy)methyl)-4-((1R,3aS,5aR,5bR,7aR,11aS,11bR,13aR,13bR)-3a-((2-((1s,4R)-1-hydroxy-4-(methylsulfonyl)cyclohexyl)ethyl)amino)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)cyclohex-3-enecarboxylate (46 mg, Yield: 34%, white solid). ¹H NMR (400MHz, CDCl₃) δ = 8.32 (dd, *J* = 2.0, 5.0 Hz, 1H), 7.86 (dd, *J* = 1.9, 7.6 Hz, 1H), 6.98 (dd, *J* = 5.0, 7.7 Hz, 1H), 6.51 (br s, 1H), 5.83-5.73 (m, 2H), 5.37-5.32 (m, 1H), 5.20-5.17 (m, 1H), 4.74-4.71 (m, 1H), 4.64-4.53 (m, 3H), 2.87-2.63 (m, 7H), 2.56-2.49 (m, 1H), 2.36-1.77 (m, 17H), 1.76-1.53 (m, 11H), 1.48-1.17 (m, 40H), 1.14-0.80 (m, 23H). LCMS: RT = 9.06 mins, (M+H) = 1166.8. HPLC Purity = 99%.

### Example 4: (S)-(Hexacosanoyloxy)methyl 1-(((3-cyanopyridin-2-yl)oxy)methyl)-4-((1R,3aS,5aR,5bR,7aR,11aS,11bR,13aR,13bR)-3a-((2-((1s,4R)-1-hydroxy-4-(methylsulfonyl)cyclohexyl)ethyl)amino)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)cyclohex-3-enecarboxylate

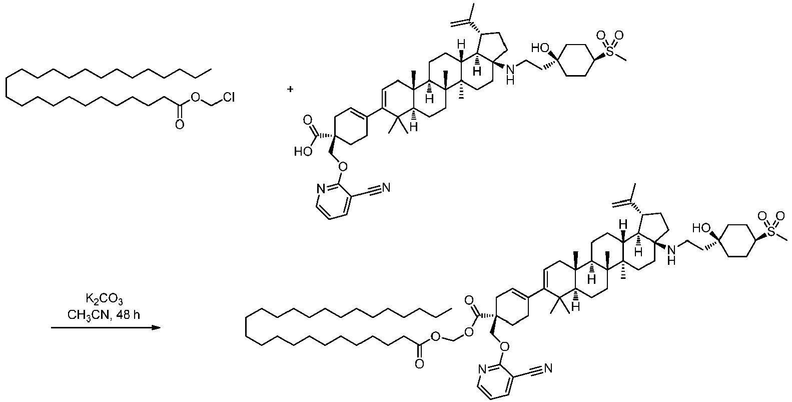

To a stirred solution of (*S*)-1-(((3-cyanopyridin-2-yl)oxy)methyl)-4-((1R,3aS,5aR,5bR,7aR,11aS,11bR,13aR,13bR)-3a-((2-((1s,4R)-1-hydroxy-4-(methylsulfonyl)cyclohexyl)ethyl)amino)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)cyclohex-3-enecarboxylic acid (100 mg, 0.115 mmol) and chloromethyl hexacosanoate (128 mg, 0.287 mmol) in CH₃CN (5 mL) and DMF (5 mL) was added potassium carbonate (79 mg, 0.575 mmol) and sodium iodide (8.61 mg, 0.057 mmol) at 0 °C. The reaction mixture was allowed to warm to 27 °C and then heated to 50 °C and stirred for 48 h. The progress of the reaction was monitored by TLC (SiO₂, 10% MeOH/DCM, Rf = 0.6, KMnO₄-active, UV-active). The reaction mixture was quenched with water (10 mL) and extracted with DCM (2 × 10 mL). The combined organic layers were washed with brine (2 × 20 mL), dried over Na₂SO₄, filtered and concentrated under reduced pressure to afford the crude product. The crude product was purified by normal phase column chromatography on neutral alumina with 0-1% MeOH/ DCM. The fractions containing product were collected and concentrated under reduced pressure to afford (S)-(hexacosanoyloxy)methyl 1-(((3-cyanopyridin-2-yl)oxy)methyl)-4-((1R,3aS,5aR,5bR,7aR,11aS,11bR,13aR,13bR)-3a-((2-((1s,4R)-1-hydroxy-4-(methylsulfonyl)cyclohexyl)ethyl)amino)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)cyclohex-3-enecarboxylate (35 mg, Yield: 23%, pale yellow solid). ¹H NMR (400MHz, CDCl₃) δ = 8.32 (dd, *J* = 2.0, 5.0 Hz, 1H), 7.86 (dd, *J* = 2.0, 7.5 Hz, 1H), 6.98 (dd, *J* = 5.0, 7.7 Hz, 1H), 5.84-5.73 (m, 2H), 5.36-5.33 (m, 1H), 5.20-5.17 (m, 1H), 4.73-4.71 (m, 1H), 4.62-4.52 (m, 3H), 3.40-3.30 (m, 1H), 2.83 (s, 3H), 2.80-2.63 (m, 4H), 2.60-2.48 (m, 1H), 2.30 (t, *J* = 7.6 Hz, 2H), 2.24 (br s, 1H), 2.19-2.14 (m, 1H), 2.13-2.05 (m, 3H), 2.04-1.87 (m, 8H), 1.87-1.85 (m, 1H), 1.76-1.55 (m, 11H), 1.51-1.36 (m, 8H), 1.35-1.17 (m, 50H), 1.16-0.99 (m, 8H), 0.96 (s, 6H), 0.93-0.82 (m, 9H). LCMS: RT = 4.72 mins, (M+H) = 1279.5. HPLC Purity = 98%.

### Example 5: (1S)-1-(Decanoyloxy)ethyl 1-(((3-cyanopyridin-2-yl)oxy)methyl)-4-((1R,3aS,5aR,5bR,7aR,11aS,11bR,13aR,13bR)-3a-((2-((1s,4R)-1-hydroxy-4-(methylsulfonyl)cyclohexyl)ethyl)amino)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)cyclohex-3-enecarboxylate

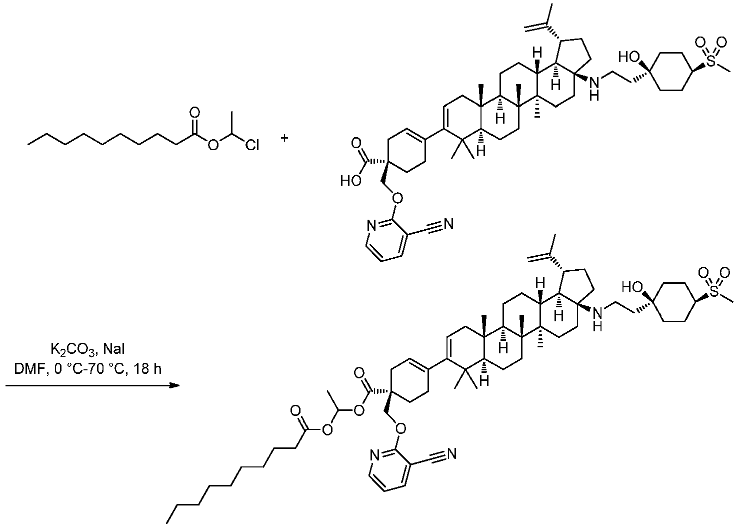

To a stirred solution of (*S*)-1-(((3-cyanopyridin-2-yl)oxy)methyl)-4-((1R,3aS,5aR,5bR,7aR,11aS,11bR,13aR,13bR)-3a-((2-((1s,4R)-1-hydroxy-4-(methylsulfonyl)cyclohexyl)ethyl)amino)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)cyclohex-3-enecarboxylic acid (100 mg, 0.115 mmol), in DMF (5 mL) was added 1-chloroethyl decanoate (81 mg, 0.345 mmol) and sodium iodide (0.344 mg, 2.298 µmol) at 27 °C under nitrogen. Then, the reaction mixture was added solid potassium carbonate (79 mg, 0.575 mmol) in one portion and it was stirred at 70 °C for 18 hr. The progress of the reaction was monitored by TLC (SiO₂, 5% MeOH/DCM, Rf = 0.2, KMnO₄-active). On completion, the reaction mixture was quenched with water (50 mL) and extracted with EtOAc (2 × 50 mL). The combined organic layers were washed with brine (50 mL), dried over Na₂SO₄, filtered and concentrated under reduced pressure to afford the crude product. The crude product was purified by normal phase column chromatography on silica gel (100-200 mesh, 0-3% MeOH/DCM). The fractions containing product were collected and concentrated under reduced pressure to afford the desired product (90 mg) along with DMF residual solvent. Previous batch product (22 mg , N66148-27-A2) was combined with this batch (90 mg) and the combined product was triturated with cold n-pentane (2 × 25 mL), dried under high vacuum and lyophilized with acetonitrile and water (1:1) for 20 h to afforded (1*S*)-1-(decanoyloxy)ethyl 1-(((3-cyanopyridin-2-yl)oxy)methyl)-4-((1R,3aS,5aR,5bR,7aR,11aS,11bR,13aR,13bR)-3a-((2-((1s,4R)-1-hydroxy-4-(methylsulfonyl)cyclohexyl)ethyl)amino)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)cyclohex-3-enecarboxylate (60 mg, Yield: 46%, white solid). ¹H NMR (400MHz,CDCl₃) δ = 8.31 (ddd, *J* = 0.9, 2.0, 5.0 Hz, 1H), 7.86 (dd, *J* = 1.9, 7.6 Hz, 1H), 7.02-6.94 (m, 1H), 6.88 (dq, *J* = 3.1, 5.4 Hz, 1H), 5.36-5.33 (m, 1H), 5.21-5.17 (m, 1H), 4.74-4.71 (m, 1H), 4.63-4.46 (m, 3H), 2.87-2.63 (m, 7H), 2.57-2.49 (m, 1H), 2.30-1.77 (m, 18H), 1.65-1.37 (m, 22H), 1.33-1.18 (m, 20H), 1.15-0.81 (m, 23H). LCMS: RT = 3.47 mins, (M+H) = 1068.7. HPLC purity = 95%, Chiral HPLC: Peak I = 48%; Peak II = 51%. Note that since the compound is a diastereomeric mixture, proton integration for alkyl region is more with reference to aromatic proton.

### Example 6: (1S)-1-(Stearoyloxy)ethyl 1-(((3-cyanopyridin-2-yl)oxy)methyl)-4-((1R,3aS,5aR,5bR,7aR,11aS,11bR,13aR,13bR)-3a-((2-((1s,4R)-1-hydroxy-4-(methylsulfonyl)cyclohexyl)ethyl)amino)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)cyclohex-3-enecarboxylate

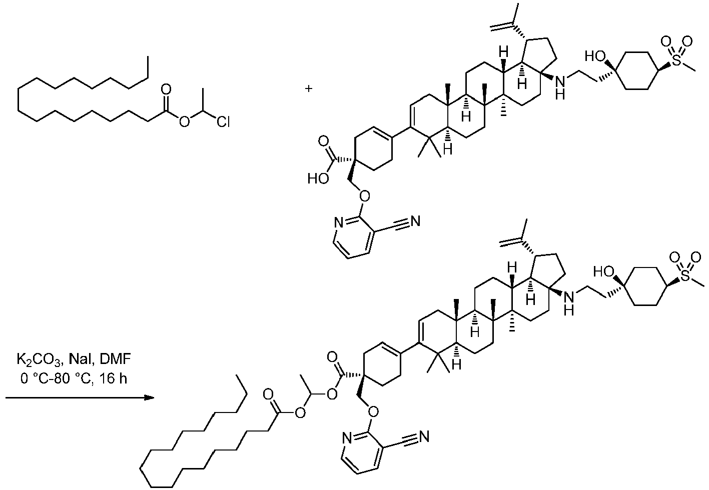

To a stirred solution of (*S*)-1-(((3-cyanopyridin-2-yl)oxy)methyl)-4-((1R,3aS,5aR,5bR,7aR,11aS,11bR,13aR,13bR)-3a-((2-((1s,4R)-1-hydroxy-4-(methylsulfonyl)cyclohexyl)ethyl)amino)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)cyclohex-3-enecarboxylic acid (100 mg, 0.115 mmol) and 1-chloroethyl stearate (140 mg, 0.402 mmol,) in DMF (5 mL) was added sodium iodide (8.61 mg, 0.057 mmol) and potassium carbonate (31.8 mg, 0.230 mmol) at 0 °C and it was slowly warmed to 27 °C. Then, the reaction mixture was heated to 80 °C and stirred for 16 hr. Progress of the reaction was monitored by TLC (SiO₂, 5% MeOH/DCM, Rf = 0.3, KMnO₄-active, UV-active). On completion of starting material, the reaction mixture was allowed to cool to 27 °C, diluted with EtOAc (30 mL) and washed with water (3 × 10 mL). The aqueous phase was separated and extracted with EtOAc (3 × 20 mL). The combined organic layers were washed with saturated brine (20 mL) and dried over anhydrous Na₂SO₄, filtered and concentrated under reduced pressure to afford the crude product as pale yellow oil. The crude compound was purified by normal phase column chromatography (SiO₂, 100-200 mesh, 0-3% MeOH/DCM). The fractions containing product were collected and concentrated under reduced pressure to afford the desired product (40 mg, off white solid). The product was triturated with cold n-pentane (2 × 25 mL), dried under high vacuum to remove DMF residual solvent impurity. It was further lyophilized with acetonitrile and water (1:1) for 20 h to afford (1*S*)-1-(stearoyloxy)ethyl 1-(((3-cyanopyridin-2-yl)oxy)methyl)-4-((1R,3aS,5aR,5bR,7aR,11aS,11bR,13aR,13bR)-3a-((2-((1s,4R)-1-hydroxy-4-(methylsulfonyl)cyclohexyl)ethyl)amino)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)cyclohex-3-enecarboxylate (27 mg, Yield: 18%, white fluffy solid). ¹H NMR (400MHz, CDCl₃) δ = 8.31 (ddd, *J* = 0.9, 2.0, 5.0 Hz, 1H), 7.86 (dd, *J* = 2.0, 7.5 Hz, 1H), 7.02-6.94 (m, 1H), 6.87 (dq, *J* = 3.1, 5.4 Hz, 1H), 5.37-5.33 (m, 1H), 5.20-5.18 (m, 1H), 4.75-4.70 (m, 1H), 4.65-4.46 (m, 3H), 2.87-2.63 (m, 7H), 2.58-2.50 (m, 1H), 2.36-1.82 (m, 18H), 1.63-1.36 (m, 28H), 1.35-1.17 (m, 33H), 1.12-0.80 (m, 24H). LCMS: RT = 4.07 mins, (M+H) = 1181.3. HPLC purity = 95%, Chiral HPLC: Peak I = 50%; Peak II = 49%. Note that since the compound is a diastereomeric mixture, proton integration for alkyl region is more with reference to aromatic proton.

### Example 7: (Decanoyloxy)methyl (R)-4-((1R,3aS,5aR,5bR,7aR,11aS,11bR,13aR,13bR)-3a-((2-(1,1-dioxidothiomorpholino)ethyl)amino)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)-1-(fluoromethyl)cyclohex-3-ene-1-carboxylate

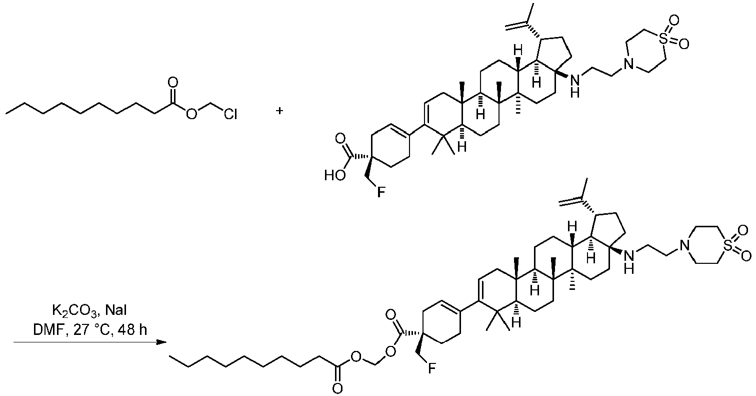

To a stirred solution of (*R*)-4-((1R,3aS,5aR,5bR,7aR,11aS,11bR,13aR,13bR)-3a-((2-(1,1-dioxidothiomorpholino)ethyl)amino)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)-1-(fluoromethyl)cyclohex-3-ene-1-carboxylic acid (200 mg, 0.275 mmol) and chloromethyldecanoate (97 mg, 0.440 mmol) in DMF (10 mL) were added sodium iodide (4.12 mg, 0.028 mmol) and potassium carbonate (190 mg, 1.375 mmol) at 0 °C. The reaction mixture was allowed to warm to 27 °C and stirred for 48 h. The progress of the reaction was monitored by TLC (SiO₂, 10% MeOH/DCM, Rf = 0.6, KMnO₄-active). On completion, the reaction mixture was quenched with ice cold water (40 mL) and extracted with EtOAc (2 × 30 mL). The combined organic layers were washed with brine (3 × 50 mL), dried over anhydrous Na₂SO₄, filtered and concentrated under reduced pressure to afford the crude product. The crude product was purified by normal phase column chromatography on neutral alumina (30-98% EtOAc/Pet.). The fractions containing product were collected and concentrated under reduced pressure to afford (decanoyloxy)methyl (*R*)-4-((1R,3aS,5aR,5bR,7aR,11aS,11bR,13aR,13bR)-3a-((2-(1,1-dioxidothiomorpholino)ethyl)amino)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)-1-(fluoromethyl)cyclohex-3-ene-1-carboxylate (82 mg, Yield: 32%, pale yellow solid). ¹H NMR (400MHz, CDCl₃) δ = 5.85-5.73 (m, 2H), 5.36-5.28 (m, 1H), 5.18-5.14 (m, 1H), 4.72-4.68 (m, 1H), 4.60-4.55 (m, 1H), 4.57-4.49 (m, 1H), 4.48-4.38 (m, 1H), 3.17-2.94 (m, 8H), 2.72-2.41 (m, 6H), 2.33 (t, *J* = 7.6 Hz, 2H), 2.25-1.71 (m, 10H), 1.64-1.46 (m, 8H), 1.44-1.15 (m, 22H), 1.14-0.77 (m, 23H). LCMS: RT = 7.71 mins, (M+H) = 911.6. HPLC Purity = 98%.

### Example 8: (R)-(Stearoyloxy)methyl 4-((1R,3aS,5aR,5bR,7aR,11aS,11bR,13aR,13bR)-3a-((2-(1,1-dioxidothiomorpholino)ethyl)amino)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)-1-(fluoromethyl)cyclohex-3-enecarboxylate

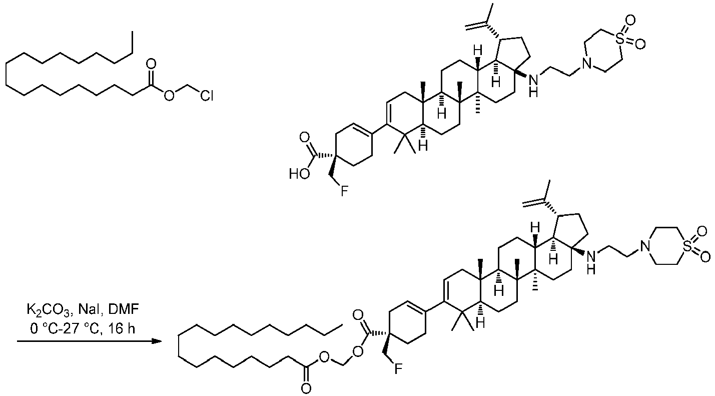

To a stirred solution of (*R*)-4-((1R,3aS,5aR,5bR,7aR,11aS,11bR,13aR,13bR)-3a-((2-(1,1-dioxidothiomorpholino)ethyl)amino)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)-1-(fluoromethyl)cyclohex-3-enecarboxylic acid (200 mg, 0.248 mmol) in DMF (10 mL) was added potassium carbonate (140 mg, 0.990 mmol), chloromethyl stearate (229 mg, 0.619 mmol) and sodium iodide (7.57 g, 0.050 mmol) at 0 °C. The reaction mixture was allowed to warm to 27 °C and stirred for 16 h. The progress of the reaction was monitored by TLC (SiO₂, 80% EtOAc/Pet. Rf = 0.3, KMnO₄-active). On completion, the reaction mixture was diluted with DCM (50 mL) and washed with water (2 × 25 mL), brine (25 mL), dried over anhydrous Na₂SO₄, filtered and concentrated under vacuum to get crude residue. The crude compound was purified by normal column chromatography on neutral Al₂O₃ with 10-40% EtOAc/Pet. The fractions containing pure product were collected and concentrated under vacuum to afford (R)-(stearoyloxy)methyl 4-((1R,3aS,5aR,5bR,7aR,11aS,11bR,13aR,13bR)-3a-((2-(1,1-dioxidothiomorpholino)ethyl)amino)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)-1-(fluoromethyl)cyclohex-3-enecarboxylate (74 mg, Yield: 29%, pale brownish gummy). ¹H NMR (400MHz, CDCl₃) δ = 5.79 (q, *J* = 5.5 Hz, 2H), 5.35-5.30 (m, 1H), 5.18-5.14 (m, 1H), 4.72-4.68 (m, 1H), 4.62-4.50 (m, 2H), 4.47-4.38 (m, 1H), 3.17-2.91 (m, 8H), 2.72-2.41 (m, 6H), 2.33 (t, *J* = 7.5 Hz, 2H), 2.27-1.71 (m, 10H), 1.66-1.53 (m, 7H), 1.46-1.36 (m, 4H), 1.35-1.16 (m, 35H), 1.13-0.76 (m, 23H). LCMS (ELSD): RT = 8.214 mins, (M+H) = 1023.60. HPLC Purity = 98%.

### Example 9: (Hexacosanoyloxy)methyl (R)-4-((1R,3aS,5aR,5bR,7aR,11aS,11bR,13aR,13bR)-3a-((2-(1,1-dioxidothiomorpholino)ethyl)amino)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)-1-(fluoromethyl)cyclohex-3-ene-1-carboxylate

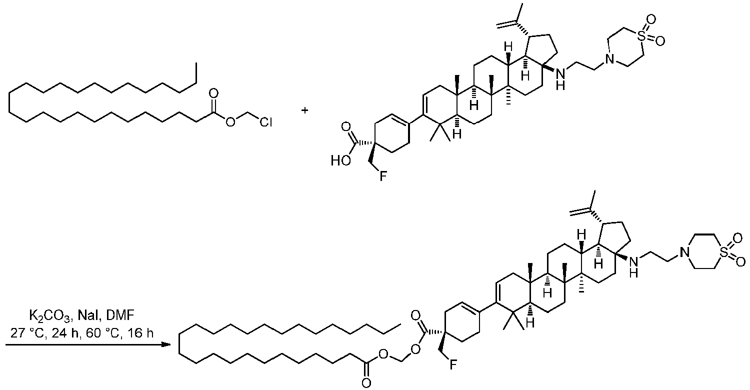

To a stirred solution of (*R*)-4-((1R,3aS,5aR,5bR,7aR,11aS,11bR,13aR,13bR)-3a-((2-(1,1-dioxidothiomorpholino)ethyl)amino)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)-1-(fluoromethyl)cyclohex-3-ene-1-carboxylic acid (250 mg, 0.344 mmol) and chloromethyl hexacosanoate (180 mg, 0.404 mmol) in DMF (10 mL) were added potassium carbonate (238 mg, 1.719 mmol) and sodium iodide (25.8 mg, 0.172 mmol) at 0 °C. The reaction mixture was allowed to warm to 27 °C and stirred for 24 h, then heated to 60 °C and stirred for 16 h. The progress of the reaction was monitored by TLC (SiO₂, 10% MeOH/DCM, Rf = 0.6, KMnO₄-active). The reaction mixture was quenched with water (50 mL) and extracted with EtOAc (2 × 50 mL). The combined organic layers were washed with brine (3 × 50 mL), dried over Na₂SO₄, filtered and concentrated under reduced pressure to afford the crude compound. The above crude compound was purified by normal phase column chromatography on neutral alumina with 50-95% EtOAC/Pet. The fractions containing product were collected and concentrated under reduced pressure to afford (hexacosanoyloxy)methyl (*R*)-4-((1R,3aS,5aR,5bR,7aR,11aS,11bR,13aR,13bR)-3a-((2-(1,1-dioxidothiomorpholino)ethyl)amino)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)-1-(fluoromethyl)cyclohex-3-ene-1-carboxylate (96 mg, Yield: 23%, pale yellow solid). ¹H NMR (400MHz, CDCl₃) δ = 5.79 (q, *J* = 5.5 Hz, 2H), 5.35-5.30 (m, 1H), 5.18-5.14 (m, 1H), 4.72-4.68 (m, 1H), 4.63-4.50 (m, 2H), 4.49-4.36 (m, 1H), 3.16-2.95 (m, 8H), 2.75-2.40 (m, 6H), 2.33 (t, *J* = 7.5 Hz, 2H), 2.25-2.04 (m, 3H), 2.00-1.72 (m, 8H), 1.68 (s, 3H), 1.64-1.53 (m, 4H), 1.46-1.37 (m, 4H), 1.37-1.16 (m, 50H), 1.15- 0.77 (m, 23H). LCMS: RT = 9.052 mins, (M+H) = 1135.8. HPLC Purity = 98%.

### Example 10: (Decanoyloxy)methyl (S)-1-(((3-cyanopyridin-2-yl)oxy)methyl)-4-((1R,3aS,5aR,5bR,7aR,11aS,11bR,13aR,13bR)-3a-((2-(1,1-dioxidothiomorpholino)ethyl)amino)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)cyclohex-3-ene-1-carboxylate

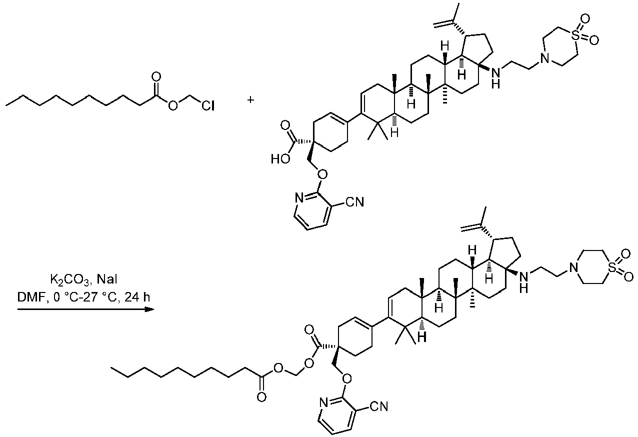

To a stirred solution of (S)-1-(((3-cyanopyridin-2-yl)oxy)methyl)-4-((1R,3aS,5aR,5bR,7aR,11aS,11bR,13aR,13bR)-3a-((2-(1,1-dioxidothiomorpholino)ethyl)amino)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)cyclohex-3-ene-1-carboxylic acid (300 mg, 0.326 mmol) and chloromethyl decanoate (108 mg, 0.488 mmol) in DMF (10 mL) was added sodium iodide (4.88 mg, 0.033 mmol) and potassium carbonate (225 mg, 1.628 mmol) at 0 °C. The reaction mixture was allowed to warm to 27 °C and stirred for 24 h. The progress of the reaction was monitored by TLC (SiO₂, 10% MeOH/DCM, Rf = 0.6, KMnO₄-active). On completion, the reaction mixture was quenched with water (20 mL) and extracted with EtOAc (2 × 20 mL). The combined organic layers were washed with brine (3 × 20 mL), dried over Na₂SO₄, filtered and concentrated under reduced pressure to give the crude compound. The crude residue was purified by normal phase column chromatography on neutral alumina (50-98% EtOAc/Pet.). The fractions containing product were collected and concentrated under reduced pressure to afford (decanoyloxy)methyl (S)-1-(((3-cyanopyridin-2-yl)oxy)methyl)-4-((1R,3aS,5aR,5bR,7aR,11as,11bR,13aR,13bR)-3a-((2-(1,1-dioxidothiomorpholino)ethyl)amino)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)cyclohex-3-ene-1-carboxylate (140 mg, Yield: 39%, yellow solid). ¹H NMR (400 MHz, CDCl₃) δ = 8.37-8.29 (m, 1H), 7.90-7.82 (m, 1H), 7.02-6.91 (m, 1H), 5.84-5.72 (m, 2H), 5.40-5.31 (m, 1H), 5.21-5.14 (m, 1H), 4.74-4.65 (m, 1H), 4.63-4.49 (m, 3H), 3.14-2.95 (m, 6H), 2.79-2.40 (m, 5H), 2.38-2.05 (m, 6H), 2.02-1.71 (m, 6H), 1.64-1.37 (m, 17H), 1.36-1.15 (m, 18H), 1.12-0.78 (m, 21H). LCMS: RT = 8.19 mins, (M+H) = 1011.6, LCMS Purity = 92%, HPLC Purity = 95%, Chiral HPLC Purity = 95%.

### Example 11: (Stearoyloxy)methyl (S)-1-(((3-cyanopyridin-2-yl)oxy)methyl)-4-((1R,3aS,5aR,5bR,7aR,11aS,11bR,13aR,13bR)-3a-((2-(1,1-dioxidothiomorpholino)ethyl)amino)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)cyclohex-3-ene-1-carboxylate

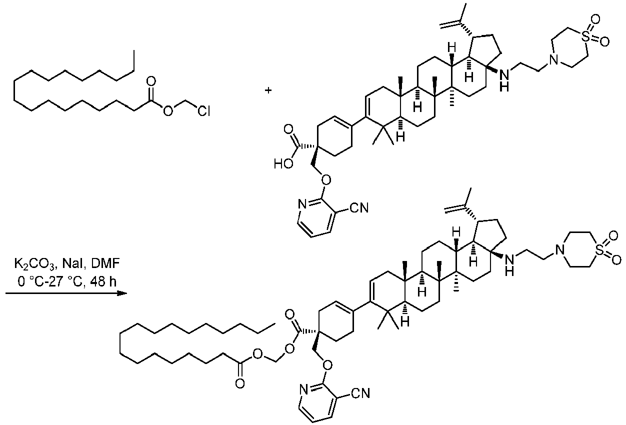

To a stirred solution of (S)-1-(((3-cyanopyridin-2-yl)oxy)methyl)-4-((1R,3aS,5aR,5bR,7aR,11aS,11bR,13aR,13bR)-3a-((2-(1,1-dioxidothiomorpholino)ethyl)amino)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)cyclohex-3-ene-1-carboxylic acid (400 mg, 0.484 mmol) in DMF (10 mL) was added chloromethyl stearate (193 mg, 0.580 mmol), potassium carbonate (334 mg, 2.418 mmol) and sodium iodide (14.50 mg, 0.097 mmol) at 27 °C. The reaction mixture was stirred at 27 °C for 48 h. The progress of the reaction was monitored by TLC (SiO₂, 10% MeOH/DCM, Rf = 0.6, KMnO₄-active). The reaction mixture was quenched with water (20 mL) and extracted with EtOAc (2 × 20 mL). The combined organic layers were washed with brine (3 × 20 mL), dried over Na₂SO₄, filtered and concentrated under reduced pressure to get the crude compound. The crude residue was purified by normal phase column chromatography on neutral alumina (0-1% MeOH/DCM). The fractions containing pure product were collected and concentrated under reduced pressure to afford (stearoyloxy)methyl (S)-1-(((3-cyanopyridin-2-yl)oxy)methyl)-4-((1R,3aS,5aR,5bR,7aR,11aS,11bR,13aR,13bR)-3a-((2-(1,1-dioxidothiomorpholino)ethyl)amino)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)cyclohex-3-ene-1-carboxylate (68 mg, Yield; 11%, pale yellow solid). ¹H NMR (400 MHz, CDCl₃) δ = 8.38-8.24 (m, 1H), 7.93-7.80 (m, 1H), 7.04-6.92 (m, 1H), 5.86-5.70 (m, 2H), 5.41-5.29 (m, 1H), 5.22-5.13 (m, 1H), 4.76-4.66 (m, 1H), 4.63-4.46 (m, 3H), 3.14-2.94 (m, 7H), 2.81-2.40 (m, 6H), 2.37-2.05 (m, 7H), 2.04-1.72 (m, 6H), 1.70-1.54 (m, 6H), 1.48-1.36 (m, 4H), 1.35-1.16 (m, 37H), 1.13-0.79 (m, 23H). LCMS: RT = 8.79 mins, (M+H) = 1124.4, LCMS Purity = 95%, HPLC Purity = 96%, Chiral HPLC Purity = 95%.

### Example 12: (Hexacosanoyloxy)methyl (S)-1-(((3-cyanopyridin-2-yl)oxy)methyl)-4-((1R,3aS,5aR,5bR,7aR,11aS,11bR,13aR,13bR)-3a-((2-(1,1-dioxidothiomorpholino)ethyl)amino)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)cyclohex-3-ene-1-carboxylate

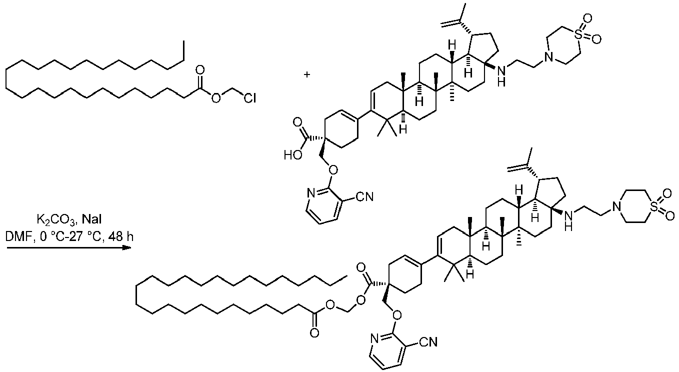

To a stirred solution of (S)-1-(((3-cyanopyridin-2-yl)oxy)methyl)-4-((1R,3aS,5aR,5bR,7aR,11aS,11bR,13aR,13bR)-3a-((2-(1,1-dioxidothiomorpholino)ethyl)amino)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)cyclohex-3-ene-1-carboxylic acid (400 mg, 0.434 mmol) and chloromethyl hexacosanoate (251 mg, 0.564 mmol) in DMF (20 mL) was added sodium iodide (6.51 mg, 0.043 mmol) and potassium carbonate (300 mg, 2.170 mmol) at 0 °C. The reaction mixture was allowed to warm to 27 °C and stirred for 48 h. The progress of the reaction was monitored by TLC (SiO₂, 10% MeOH/DCM, Rf = 0.6, KMnO₄-active). The reaction mixture was quenched with water (25 mL) and extracted with EtOAc (2 × 25 mL). The combined organic layers were washed with brine (3 × 30 mL), dried over Na₂SO₄, filtered and concentrated under reduced pressure to afford the crude compound. The crude compound was purified by normal phase column chromatography on neutral alumina (50-98% EtOAc/Pet.). The fractions containing product were collected and concentrated under reduced pressure to afford (hexacosanoyloxy)methyl (S)-1-(((3-cyanopyridin-2-yl)oxy)methyl)-4-((1R,3aS,5aR,5bR,7aR,11aS,11bR,13aR,13bR)-3a-((2-(1,1-dioxidothiomorpholino)ethyl)amino)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)cyclohex-3-ene-1-carboxylate (72 mg, Yield: 13%, yellow solid). ¹H NMR (400 MHz, CDCl₃) δ = 8.38-8.28 (m, 1H), 7.86 (dd, *J* = 2.0, 7.5 Hz, 1H), 7.02-6.93 (m, 1H), 5.84-5.72 (m, 2H), 5.37-5.33 (m, 1H), 5.24-5.15 (m, 1H), 4.76-4.67 (m, 1H), 4.64-4.51 (m, 3H), 3.14-2.95 (m, 7H), 2.74-2.52 (m, 5H), 2.50-2.41 (m, 1H), 2.37-2.05 (m, 7H), 2.02-1.52 (m, 14H), 1.48-1.38 (m, 3H), 1.37-1.16 (m, 52H), 1.14-1.01 (m, 7H), 1.00-0.93 (m, 6H), 0.93-0.81 (m, 9H). LCMS: RT = 8.77 mins, (M+H) = 1236.4, LCMS Purity = 97%, HPLC Purity = 97%, Chiral HPLC Purity = 95%.

### Example 13: 2-(Dimethylamino)-2-oxoethyl (S)-1-(((3-cyanopyridin-2-yl)oxy)methyl)-4-((1R,3aS,5aR,5bR,7aR,11aS,11bR,13aR,13bR)-3a-((2-((1s,4R)-1-hydroxy-4-(methylsulfonyl)cyclohexyl)ethyl)amino)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)cyclohex-3-ene-1-carboxylate (not according to the present invention)

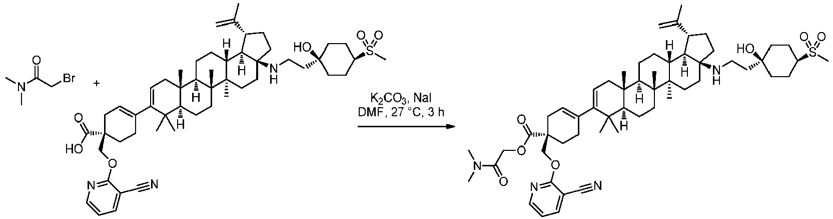

To a stirred solution of (S)-1-(((3-cyanopyridin-2-yl)oxy)methyl)-4-((1R,3aS,5aR,5bR,7aR,11aS,11bR,13aR,13bR)-3a-((2-((1s,4R)-1-hydroxy-4-(methylsulfonyl)cyclohexyl)ethyl)amino)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)cyclohex-3-ene-1-carboxylic acid (200 mg, 0.230 mmol) and 2-bromo-N,N-dimethylacetamide (57.2 mg, 0.345 mmol) in DMF (10 mL) was added potassium carbonate (79 mg, 0.575 mmol) and sodium iodide (17.22 mg, 0.115 mmol) at 27 °C. The reaction mixture was stirred at 27 °C for 3 h. Progress of the reaction was monitored by TLC (SiO₂, 5% MeOH/DCM, Rf = 0.2, UV-active, KMnO₄-active). The reaction mixture was diluted with EtOAc (30 mL) and water (20 mL). The aqueous phase was separated and extracted with EtOAc (2 × 20 mL). The combined organic layers were washed with ice cold water (4 × 20 mL), dried over Na₂SO₄, filtered and concentrated under reduced pressure to give the crude compound as pale yellow solid. The crude compound was triturated with cold Et₂O (2×5 mL) and dried under high vacuum. It was further diluted with acetonitrile (15 mL) and water (15 mL), the solution was frozen and lyophilised for 20 h to afford 2-(dimethylamino)-2-oxoethyl (S)-1-(((3-cyanopyridin-2-yl)oxy)methyl)-4-((1R,3aS,5aR,5bR,7aR,11aS,11bR,13aR,13bR)-3a-((2-((1s,4R)-1-hydroxy-4-(methylsulfonyl)cyclohexyl)ethyl)amino)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)cyclohex-3-ene-1-carboxylate (133 mg, Yield: 58%, off white solid). ¹H NMR (400 MHz, CDCl₃) δ = 8.38-8.28 (m, 1H), 7.85 (dd, J = 2.0, 7.5 Hz, 1H), 7.03-6.89 (m, 1H), 5.37-5.32 (m, 1H), 5.24-5.16 (m, 1H), 4.85-4.69 (m, 3H), 4.68-4.55 (m, 3H), 2.97 (s, 3H), 2.92 (s, 3H), 2.86-2.66 (m, 7H), 2.59-2.45 (m, 1H), 2.40-2.23 (m, 2H), 2.22-1.76 (m, 13H), 1.75-1.57 (m, 7H), 1.54-1.17 (m, 14H), 1.13-0.76 (m, 20H). LCMS: RT = 6.47 mins, (M+H) = 955.6, LCMS Purity = 98%, HPLC Purity = 98%, Chiral HPLC Purity = 98%.

### Example 14:: (Octanoyloxy)methyl (S)-1-(((3-cyanopyridin-2-yl)oxy)methyl)-4-((1R,3aS,5aR,5bR,7aR,11aS,11bR,13aR,13bR)-3a-((2-((1s,4R)-1-hydroxy-4-(methylsulfonyl)cyclohexyl)ethyl)amino)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)cyclohex-3-ene-1-carboxylate

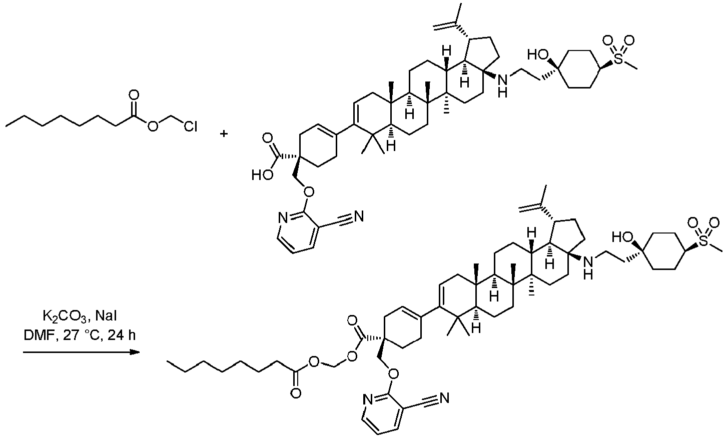

To a stirred solution of (*S*)-1-(((3-cyanopyridin-2-yl)oxy)methyl)-4-((1R,3aS,5aR,5bR,7aR,11aS,11bR,13aR,13bR)-3a-((2-((1s,4R)-1-hydroxy-4-(methylsulfonyl)cyclohexyl)ethyl)amino)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)cyclohex-3-ene-1-carboxylic acid (N68486-7-A2, 200 mg, 0.230 mmol) and chloromethyl octanoate (N68486-19-A2, 53.1 mg, 0.276 mmol) in DMF (10 mL) was added potassium carbonate (159 mg, 1.149 mmol) followed by sodium iodide (6.89 mg, 0.046 mmol) at 0 °C. The resulting reaction mixture was allowed to warm to 27 °C and stirred for 24 hr. The progress of the reaction was monitored by TLC (SiO₂, 5% MeOH/DCM, Rf = 0.4, KMnO₄-active). The reaction mixture was quenched with water (20 mL) and extracted with EtOAc (2 × 30 mL). The combined organic layers were washed with brine (3 × 20 mL), dried over Na₂SO₄, filtered and concentrated under reduced pressure to afford the crude residue as sticky oil. The crude compound was triturated with n-pentane (3 × 10 mL) and dried under high vacuum to afford (octanoyloxy)methyl (S)-1-(((3-cyanopyridin-2-yl)oxy)methyl)-4-((1R,3aS,5aR,5bR,7aR,11aS,11bR,13aR,13bR)-3a-((2-((1s,4R)-1-hydroxy-4-(methylsulfonyl)cyclohexyl)ethyl)amino)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)cyclohex-3-ene-1-carboxylate (N68486-20-A1, 145 mg, Yield: 59%, pale grey solid). ¹H NMR (400MHz, CDCl₃) δ = 8.32 (dd, *J* = 2.0, 5.0 Hz, 1H), 7.86 (dd, *J* = 2.0, 7.5 Hz, 1H), 6.98 (dd, *J* = 5.0, 7.5 Hz, 1H), 5.80-5.75 (m, 2H), 5.36-5.32 (m, 1H), 5.19-5.17 (m, 1H), 4.74-4.72 (m, 1H), 4.61-4.55 (m, 3H), 2.86-2.65 (m, 6H), 2.58-2.52 (m, 1H), 2.34-1.78 (m, 19H), 1.66-1.20 (m, 30H), 1.14-1.02 (m, 7H), 0.96 (s, 6H), 0.92-0.82 (m, 9H). LCMS: RT = 7.63 mins, (M+H) = 1026.6, LCMS Purity = 96%, HPLC Purity = 97%, Chiral HPLC Purity = 99%.

### Example 15: (Dodecanoyloxy)methyl (S)-1-(((3-cyanopyridin-2-yl)oxy)methyl)-4-((1R,3aS,5aR,5bR,7aR,11aS,11bR,13aR,13bR)-3a-((2-((1s,4R)-1-hydroxy-4-(methylsulfonyl)cyclohexyl)ethyl)amino)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)cyclohex-3-ene-1-carboxylate

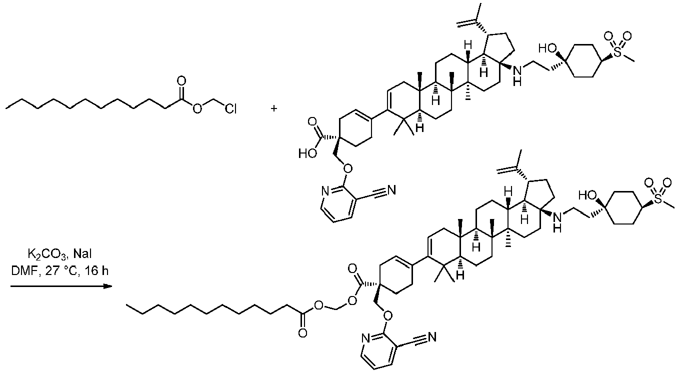

To a stirred solution of (S)-1-(((3-cyanopyridin-2-yl)oxy)methyl)-4-((1R,3aS,5aR,5bR,7aR,11aS,11bR,13aR,13bR)-3a-((2-((1s,4R)-1-hydroxy-4-(methylsulfonyl)cyclohexyl)ethyl)amino)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)cyclohex-3-ene-1-carboxylic acid (N67808-42-A3, 150 mg, 0.167 mmol) and chloromethyl dodecanoate (N67709-82-A2, 49.9 mg, 0.201 mmol) in DMF (5 mL) was added potassium carbonate (116 mg, 0.836 mmol) followed by sodium iodide (5.01 mg, 0.033 mmol) at 0 °C. The resulting reaction mixture was allowed to warm to 27 °C and stirred for 16 hr. The progress of the reaction was monitored by TLC (SiO₂, 5% MeOH/DCM, Rf = 0.5, KMnO₄-active). The reaction mixture was quenched with water (20 mL) and extracted with EtOAc (2 × 20 mL). The combined organic layers were washed with brine (3 × 20 mL), dried over Na₂SO₄, filtered and concentrated under reduced pressure to afford the crude residue. The crude compound was triturated with *n-*pentane (3 × 15 mL) and dried under high vacuum to afford (dodecanoyloxy)methyl (S)-1-(((3-cyanopyridin-2-yl)oxy)methyl)-4-((1R,3aS,5aR,5bR,7aR,11aS,11bR,13aR,13bR)-3a-((2-((1s,4R)-1-hydroxy-4-(methylsulfonyl)cyclohexyl)ethyl)amino)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)cyclohex-3-ene-1-carboxylate (N67709-99-A1, 106 mg, Yield: 55%, off-white solid). ¹H NMR (400 MHz, CDCl₃) δ = 8.32 (dd, *J* = 2.0, 5.0 Hz, 1H), 7.86 (dd, *J* = 2.0, 7.5 Hz, 1H), 6.98 (dd, *J* = 5.0, 7.5 Hz, 1H), 5.81-5.76 (m, 2H), 5.37-5.33 (m, 1H), 5.20-5.18 (m, 1H), 4.74-4.71 (m, 1H), 4.61-4.54 (m, 3H), 2.86-2.65 (m, 7H), 2.58-2.50 (m, 1H), 2.34-1.78 (m, 18H), 1.65-1.38 (m, 17H), 1.36-1.18 (m, 22H), 1.14-0.82 (m, 23H). LCMS: RT = 15.43 mins, (M+H) = 1082.6, LCMS Purity = 95%, HPLC Purity = 95%, Chiral HPLC Purity = 97%.

### Example 16: Acetoxymethyl (R)-4-((1R,3aS,5aR,5bR,7aR,11aS,11bR,13aR,13bR)-3a-((2-(1,1-dioxidothiomorpholino)ethyl)amino)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)-1-(fluoromethyl)cyclohex-3-ene-1-carboxylate

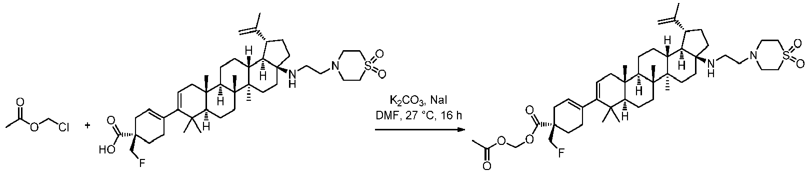

To a stirred solution of (R)-4-((1R,3aS,5aR,5bR,7aR,11aS,11bR,13aR,13bR)-3a-((2-(1,1-dioxidothiomorpholino)ethyl)amino)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)-1-(fluoromethyl)cyclohex-3-ene-1-carboxylic acid (150 mg, 0.206 mmol) in DMF (10 mL) was added chloromethyl acetate (26.9 mg, 0.248 mmol), K₂CO₃ (143 mg, 1.032 mmol) followed by sodium iodide (6.18 mg, 0.041 mmol) at 27 °C. The reaction mixture was stirred at 27 °C for 16 hr. The progress of the reaction was monitored by TLC (SiO₂, 5% MeOH/DCM, Rf = 0.3, KMnO₄-active). On completion, the reaction mixture was diluted with water (20 mL) and EtOAc (50 mL). The aqueous layer was extracted with EtOAc (2 × 50 mL). The combined organic layers were washed with brine (3 × 20 mL), dried over Na₂SO₄, filtered and concentrated under reduced pressure to give the crude residue. The above crude product was triturated with n-pentane (3 × 10 mL) and dried under high vacuum to afford acetoxymethyl (R)-4-((1R,3aS,5aR,5bR,7aR,11aS,11bR,13aR,13bR)-3a-((2-(1,1-dioxidothiomorpholino)ethyl)amino)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)-1-(fluoromethyl)cyclohex-3-ene-1-carboxylate (100 mg, Yield: 60%, off-white solid). ¹H NMR (400 MHz, CDCl₃) δ = 5.80-5.76 (m, 2H), 5.34-5.30 (m, 1H), 5.18-5.15 (m, 1H), 4.72-4.68 (m, 1H), 4.60-4.40 (m, 3H), 3.10-2.98 (m, 8H), 2.68-2.52 (m, 5H), 2.49-2.42 (m, 1H), 2.24-2.18 (m, 1H), 2.13-2.08 (m, 4H), 2.04-1.90 (m, 3H), 1.86-1.72 (m, 4H), 1.69-1.64 (m, 3H), 1.58-1.52 (m, 3H), 1.47-1.38 (m, 6H), 1.34-1.19 (m, 7H), 1.10-1.01 (m, 7H), 0.96 (s, 6H), 0.90 (s, 3H), 0.84 (m, 3H). LCMS: RT = 6.672 mins, (M+H) = 799.4, LCMS Purity = 99%, HPLC = 96%, Chiral HPLC = 94%.

### Example 17: (Butyryloxy)methyl (R)-4-((1R,3aS,5aR,5bR,7aR,11aS,11bR,13aR,13bR)-3a-((2-(1,1-dioxidothiomorpholino)ethyl)amino)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)-1-(fluoromethyl)cyclohex-3-ene-1-carboxylate

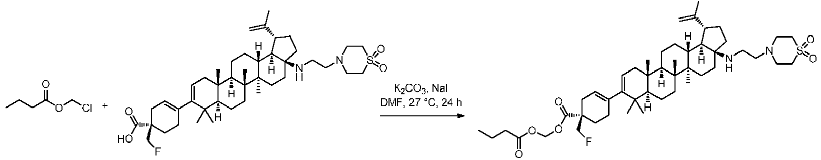

To a stirred solution of (R)-4-((1R,3aS,5aR,5bR,7aR,11aS,11bR,13aR,13bR)-3a-((2-(1,1-dioxidothiomorpholino)ethyl)amino)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)-1-(fluoromethyl)cyclohex-3-ene-1-carboxylic acid (200 mg, 0.275 mmol) in DMF (10 mL) was added chloromethyl butyrate (45.1 mg, 0.330 mmol), K₂CO₃ (190 mg, 1.375 mmol) followed by sodium iodide (8.25 mg, 0.055 mmol) at 27 °C. The reaction mixture was stirred at 27 °C for 24 hr. The progress of the reaction was monitored by TLC (SiO₂, 5% MeOH/DCM, Rf = 0.6, KMnO₄-active). The reaction mixture was quenched with water (20 mL) and extracted with EtOAc (2 × 20 mL). The combined organic layers were washed with brine (3 × 20 mL), dried over Na₂SO₄, filtered and concentrated under reduced pressure to afford the crude residue. The above crude compound was triturated with n-pentane (4 × 10 mL) and dried under high vacuum to afford (R)-4-((1R,3aS,5aR,5bR,7aR,11aS,11bR,13aR,13bR)-3a-((2-(1,1-dioxidothiomorpholino)ethyl)amino)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)-1-(fluoromethyl)cyclohex-3-ene-1-carboxylate (180 mg, Yield: 79%, pale yellow solid). ¹H NMR (400 MHz, CDCl₃) δ = 5.82-5.78 (m, 2H), 5.34-5.30 (m, 1H), 5.18-5.15 (m, 1H), 4.71-4.69 (m, 1H), 4.60-4.52 (m, 2H), 4.46-4.40 (m, 1H), 3.09-2.99 (m, 8H), 2.71-2.42 (m, 6H), 2.32 (t, *J* = 7.4 Hz, 2H), 2.22-1.72 (m, 10H), 1.69-1.54 (m, 9H), 1.46-1.18 (m, 10H), 1.10-1.01 (m, 7H), 0.97-0.83 (m, 15H). LCMS: RT = 7.369 mins, (M+H) = 827.4, Purity = 99%, HPLC Purity = 98%, Chiral HPLC Purity = 95%. **Example 18:** (Hexanoyloxy)methyl (R)-4-((1R,3aS,5aR,5bR,7aR,11as,11bR,13aR,13bR)-3a-((2-(1,1-dioxidothiomorpholino)ethyl)amino)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)-1-(fluoromethyl)cyclohex-3-ene-1-carboxylate

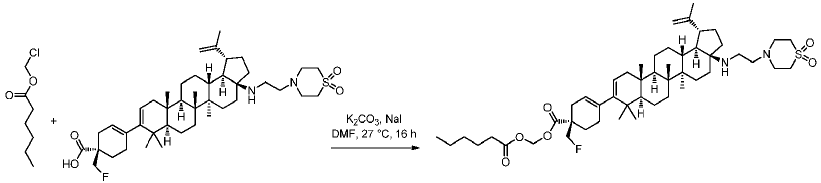

To a stirred solution of (R)-4-((1R,3aS,5aR,5bR,7aR,11aS,11bR,13aR,13bR)-3a-((2-(1,1-dioxidothiomorpholino)ethyl)amino)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)-1-(fluoromethyl)cyclohex-3-ene-1-carboxylic acid (100 mg, 0.138 mmol) and chloromethyl hexanoate (34.0 mg, 0.206 mmol) in DMF (5 mL) was added K₂CO₃ (95 mg, 0.688 mmol) followed by sodium iodide (4.12 mg, 0.028 mmol) at 0 °C. The resulting reaction mixture was allowed to warm to 27 °C and stirred for 16 hr. The progress of the reaction was monitored by TLC (SiO₂, 5% MeOH/DCM, Rf = 0.5, KMnO₄-active). The reaction mixture was quenched with water (20 mL) and extracted with EtOAc (2 × 30 mL). The combined organic layers were washed with brine (3 × 20 mL), dried over Na₂SO₄, filtered and concentrated under reduced pressure to give the crude residue. The crude compound was triturated with cold n-pentane (3 × 15 mL) and dried under high vacuum to the pure ccompound (N69114-25-A1, 60 mg) as off-white solid. *Note:* This reaction was performed in another batch (N69114-24). Both N69114-24-A1 (30 mg) and N69114-25-A1 (60 mg) batches were blended in N69114-26 (90 mg, 0.105 mmol) and dissolved in ACN and water (1:3, 40 mL). The solution was freezed and lyophilized for 16 hr to afford (hexanoyloxy)methyl (R)-4-((1R,3aS,5aR,5bR,7aR,11aS,11bR,13aR,13bR)-3a-((2-(1,1-dioxidothiomorpholino)ethyl)amino)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)-1-(fluoromethyl)cyclohex-3-ene-1-carboxylate (87 mg, Yield: 96%, off-white solid). ¹H NMR (400 MHz, CDCl₃) δ = 5.82-5.77 (m, 2H), 5.34-5.30 (m, 1H), 5.18-5.15 (m, 1H), 4.72-4.68 (m, 1H), 4.60-4.40 (m, 3H), 3.10-2.98 (m, 8H), 2.70-2.42 (m, 6H), 2.33 (t, *J* = 7.4 Hz, 2H), 2.22-1.54 (m, 20H), 1.45-1.20 (m, 13H), 1.10-0.83 (m, 22H). LCMS: RT = 7.280 mins, (M+H) = 855.4, Purity = 99%, HPLC Purity = 97%, Chiral HPLC Purity = 97%.

### Example 19: (Octanoyloxy)methyl (R)-4-((1R,3aS,5aR,5bR,7aR,11aS,11bR,13aR,13bR)-3a-((2-(1,1-dioxidothiomorpholino)ethyl)amino)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)-1-(fluoromethyl)cyclohex-3-ene-1-carboxylate

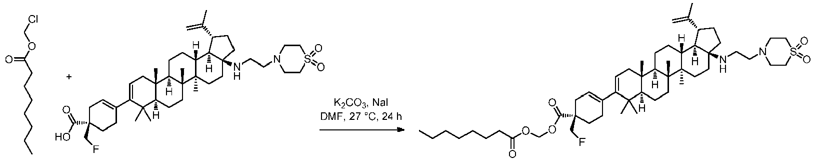

To a stirred solution of (R)-4-((1R,3aS,5aR,5bR,7aR,11aS,11bR,13aR,13bR)-3a-((2-(1,1-dioxidothiomorpholino)ethyl)amino)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)-1-(fluoromethyl)cyclohex-3-ene-1-carboxylic acid (150 mg, 0.206 mmol) in DMF (10 mL) was added chloromethyl octanoate (47.7 mg, 0.248 mmol), K₂CO₃ (143 mg, 1.032 mmol) followed by sodium iodide (6.18 mg, 0.041 mmol) at 27 °C. The reaction mixture was allowed to warm to 27 °C and stirred for 24 hr. The progress of the reaction was monitored by TLC (SiO₂, 5% MeOH/DCM, Rf = 0.3, KMnO₄-active). On completion, the reaction mixture was diluted with water (20 mL) and EtOAc (50 mL). The aqueous layer was extracted with EtOAc (2 × 50 mL). The combined organic layers were washed with brine (3 × 20 mL), dried over Na₂SO₄, filtered and concentrated under reduced pressure to afford the crude residue as pale-yellow gummy oil. The crude compound was triturated with *n*-pentane (3 × 10 mL) and dried under high vacuum to afford an off-white solid. The obtained solid was further dissolved in minimum acetonitrile and water (1:1, 10 mL) and lyophilised for 20 h to afford (octanoyloxy)methyl (R)-4-((1R,3aS,5aR,5bR,7aR,11aS,11bR,13aR,13bR)-3a-((2-(1,1-dioxidothiomorpholino)ethyl)amino)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)-1-(fluoromethyl)cyclohex-3-ene-1-carboxylate (75 mg, Yield: 40%, off-white solid). ¹H NMR (400MHz, CDCl₃) δ = 5.81-5.77 (m, 2H), 5.33-5.30 (m, 1H), 5.18-5.15 (m, 1H), 4.71-4.69 (m, 1H), 4.60-4.40 (m, 3H), 3.10-3.01 (m, 8H), 2.68-2.42 (m, 6H), 2.33 (t, *J* = 7.5 Hz, 2H), 2.22-1.73 (m, 11H), 1.69-1.54 (m, 8H), 1.46-1.39 (m, 5H), 1.35-1.20 (m, 13H), 1.08-1.01 (m, 7H), 0.97-0.93 (m, 6H), 0.91-0.84 (m, 9H). LCMS: RT = 7.77 mins, (M+H) = 883.5, LCMS Purity = 99%, HPLC = 99%, Chiral HPLC = 97%.

### Example 20: (Dodecanoyloxy)methyl (R)-4-((1R,3aS,5aR,5bR,7aR,11aS,11bR,13aR,13bR)-3a-((2-(1,1-dioxidothiomorpholino)ethyl)amino)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)-1-(fluoromethyl)cyclohex-3-ene-1-carboxylate

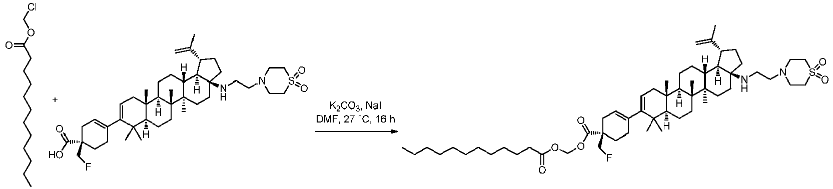

To a stirred solution of (R)-4-((1R,3aS,5aR,5bR,7aR,11aS,11bR,13aR,13bR)-3a-((2-(1,1-dioxidothiomorpholino)ethyl)amino)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)-1-(fluoromethyl)cyclohex-3-ene-1-carboxylic acid (150 mg, 0.206 mmol) and chloromethyl dodecanoate (61.6 mg, 0.248 mmol) in DMF (10 mL) was added potassium carbonate (143 mg, 1.032 mmol) followed by sodium iodide (6.18 mg, 0.041 mmol) at 0 °C. The resulting reaction mixture was allowed to warm to 27 °C and stirred for 16 hr. The progress of the reaction was monitored by TLC (SiO₂, 5% MeOH/DCM, Rf = 0.5, KMnO₄-active). The reaction mixture was quenched with water (20 mL) and extracted with EtOAc (2 × 20 mL). The combined organic layers were washed with brine (3 × 20 mL), dried over Na₂SO₄, filtered and concentrated under reduced pressure to get the crude residue. The crude compound was triturated with cold n-pentane (3 × 15 mL) and dried under high vacuum to afford (dodecanoyloxy)methyl (R)-4-((1R,3aS,5aR,5bR,7aR,11aS,11bR,13aR,13bR)-3a-((2-(1,1-dioxidothiomorpholino)ethyl)amino)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)-1-(fluoromethyl)cyclohex-3-ene-1-carboxylate (75 mg, Yield: 38%, off-white solid). ¹H NMR (400 MHz, CDCl₃) δ = 5.81-5.77 (m, 2H), 5.33-5.30 (m, 1H), 5.17-5.14 (m, 1H), 4.71-4.69 (m, 1H), 4.60-4.57 (m, 1H), 4.56-4.40 (m, 2H), 3.09-3.01 (m, 8H), 2.68-2.42 (m, 6H), 2.33 (t, *J* = 7.6 Hz, 2H), 2.20-1.73 (m, 11H), 1.69-1.54 (m, 9H), 1.46-1.39 (m, 6H), 1.34-1.21 (m, 20H), 1.08-0.84 (m, 21H). LCMS: RT = 8.259 mins, (M+H) = 939.6, Purity = 98%, HPLC Purity = 98%, Chiral HPLC Purity = 90%.

### Example 21: (Isobutyryloxy)methyl (R)-4-((1R,3aS,5aR,5bR,7aR,11aS,11bR,13aR,13bR)-3a-((2-(1,1-dioxidothiomorpholino)ethyl)amino)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)-1-(fluoromethyl)cyclohex-3-ene-1-carboxylate

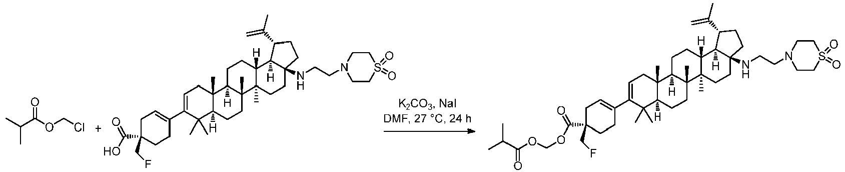

To a stirred solution of (R)-4-((1R,3aS,5aR,5bR,7aR,11aS,11bR,13aR,13bR)-3a-((2-(1,1-dioxidothiomorpholino)ethyl)amino)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)-1-(fluoromethyl)cyclohex-3-ene-1-carboxylic acid (150 mg, 0.206 mmol) in DMF (10 mL) was added chloromethyl isobutyrate (106 mg, 0.619 mmol), K₂CO₃ (143 mg, 1.032 mmol) followed by sodium iodide (6.18 mg, 0.041 mmol) at 27 °C. The reaction mixture was stirred at 27 °C for 24 hr. The progress of the reaction was monitored by TLC (SiO₂, 5% MeOH/DCM, Rf = 0.6, KMnO₄-active). The reaction mixture was quenched with water (20 mL) and extracted with EtOAc (2 × 20 mL). The combined organic layers were washed with brine (3 × 20 mL), dried over Na₂SO₄, filtered and concentrated under reduced pressure to afford the crude residue. The crude compound was triturated with n-pentane (4 × 10 mL) and dried under high vacuum to afford (isobutyryloxy)methyl (R)-4-((1R,3aS,5aR,5bR,7aR,11aS,11bR,13aR,13bR)-3a-((2-(1,1-dioxidothiomorpholino)ethyl)amino)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)-1-(fluoromethyl)cyclohex-3-ene-1-carboxylate (110 mg, Yield: 64%, pale yellow solid). ¹H NMR (400 MHz, CDCl₃) δ = 5.82-5.78 (m, 2H), 5.34-5.30 (m, 1H), 5.18-5.15 (m, 1H), 4.72-4.68 (m, 1H), 4.61-4.52 (m, 2H), 4.48-4.40 (m, 1H), 3.10-2.99 (m, 8H), 2.68-2.42 (m, 7H), 2.22-1.72 (m, 11H), 1.70-1.62 (m, 3H), 1.58-1.53 (m, 4H), 1.46-1.21 (m, 9H), 1.17 (d, *J* = 7.1 HZ, 6H), 1.10-1.01 (m, 7H), 0.98-0.93 (m, 6H), 0.92-0.88 (m, 3H), 0.86-0.83 (m, 3H). LCMS: RT = 6.947 mins, (M+H) = 827.4, Purity = 99%, HPLC Purity = 98%, Chiral HPLC Purity = 96%.

### Example 22: Acetoxymethyl (S)-1-(((3-cyanopyridin-2-yl)oxy)methyl)-4-((1R,3aS,5aR,5bR,7aR,11aS,11bR,13aR,13bR)-3a-((2-((1s,4R)-1-hydroxy-4-(methylsulfonyl)cyclohexyl)ethyl)amino)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)cyclohex-3-ene-1-carboxylate

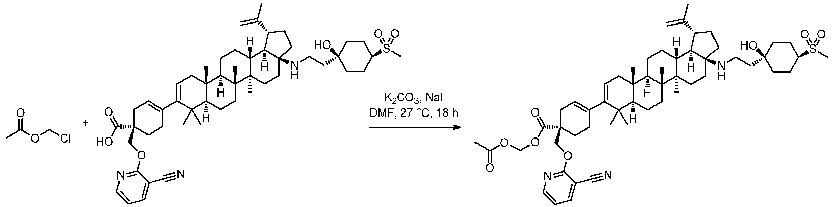

To a stirred solution of (S)-1-(((3-cyanopyridin-2-yl)oxy)methyl)-4-((1R,3aS,5aR,5bR,7aR,11aS,11bR,13aR,13bR)-3a-((2-((1s,4R)-1-hydroxy-4-(methylsulfonyl)cyclohexyl)ethyl)amino)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)cyclohex-3-enecarboxylic acid (200 mg, 0.230 mmol), in DMF (5 mL) were added chloromethyl acetate (29.9 mg, 0.276 mmol), sodium iodide (0.689 mg, 4.60 µmol) and potassium carbonate (159 mg, 1.149 mmol) at 27 °C under nitrogen atmosphere. The reaction mixture was stirred at 27 °C for 18 hr. The progress of the reaction was monitored by TLC (SiO₂, 5% MeOH/DCM, Rf = 0.2, KMnO₄-active & UV-active). On completion, the reaction mixture was poured into ice water (50 mL) and extracted with EtOAc (2 × 50 mL). The combined organic layers were washed with brine (2 × 50 mL), dried over Na₂SO₄, filtered and concentrated under reduced pressure to give the crude residue. The crude compound was triturated with chilled n-pentane (3 × 20 mL), filtered and dried under high vacuum to afford acetoxymethyl (S)-1-(((3-cyanopyridin-2-yl)oxy)methyl)-4-((1R,3aS,5aR,5bR,7aR,11aS,11bR,13aR,13bR)-3a-((2-((1s,4R)-1-hydroxy-4-(methylsulfonyl)cyclohexyl)ethyl)amino)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)cyclohex-3-ene-1-carboxylate (135 mg, Yield: 59%, brown colour solid). ¹H NMR (400 MHz, CDCl₃) δ = 8.32 (dd, *J* = 2.0, 5.0 Hz, 1H), 7.86 (dd, *J* = 2.0, 7.5 Hz, 1H), 6.98 (dd, *J* = 5.0, 7.6 Hz, 1H), 5.80-5.75 (m, 2H), 5.37-5.33 (m, 1H), 5.19-5.16 (m, 1H), 4.74-4.71 (m, 1H), 4.61-4.55 (m, 3H), 2.85-2.65 (m, 6H), 2.57-2.49 (m, 1H), 2.28-2.02 (m, 9H), 2.00-1.76 (m, 9H), 1.69-1.36 (m, 18H), 1.34-1.18 (m, 7H), 1.08-1.01 (m, 6H), 0.99-0.94 (m, 6H), 0.93-0.88 (m, 3H), 0.86-0.82 (m, 3H). LCMS: RT = 6.82 mins, (M+H) = 942.4, Purity = 95%, HPLC Purity = 94%.

### Example 23: (Butyryloxy)methyl (S)-1-(((3-cyanopyridin-2-yl)oxy)methyl)-4-((1R,3aS,5aR,5bR,7aR,11aS,11bR,13aR,13bR)-3a-((2-((1s,4R)-1-hydroxy-4-(methylsulfonyl)cyclohexyl)ethyl)amino)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)cyclohex-3-ene-1-carboxylate

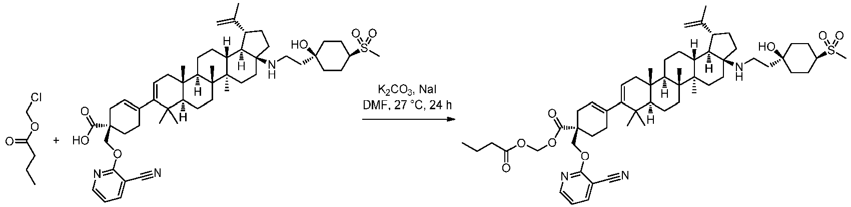

To a stirred solution of (S)-1-(((3-cyanopyridin-2-yl)oxy)methyl)-4-((1R,3aS,5aR,5bR,7aR,11aS,11bR,13aR,13bR)-3a-((2-((1s,4R)-1-hydroxy-4-(methylsulfonyl)cyclohexyl)ethyl)amino)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)cyclohex-3-ene-1-carboxylic acid (200 mg, 0.230 mmol) and chloromethyl butyrate (37.7 mg, 0.276 mmol) in DMF (10 mL) was added potassium carbonate (159 mg, 1.149 mmol) followed by sodium iodide (6.89 mg, 0.046 mmol) at 0 °C. The resulting reaction mixture was allowed to warm to 27 °C and stirred for 24 hr. The progress of the reaction was monitored by TLC (SiO₂, 5% MeOH/ DCM, Rf = 0.4, KMnO₄-active). The reaction mixture was quenched with water (20 mL) and extracted with EtOAc (2 × 30 mL). The combined organic layers were washed with brine (3 × 20 mL), dried over Na₂SO₄, filtered and concentrated under reduced pressure to give the crude residue. The crude compound was triturated with n-pentane (3 × 10 mL) and dried under high vacuum to afford (butyryloxy)methyl (S)-1-(((3-cyanopyridin-2-yl)oxy)methyl)-4-((1R,3aS,5aR,5bR,7aR,11aS,11bR,13aR,13bR)-3a-((2-((1s,4R)-1-hydroxy-4-(methylsulfonyl)cyclohexyl)ethyl)amino)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)cyclohex-3-ene-1-carboxylate (107 mg, Yield: 46%, off-white solid). ¹H NMR (400 MHz, CDCl₃) δ = 8.32 (dd, *J* = 2.0, 5.0 Hz, 1H), 7.86 (dd, *J* = 2.0, 7.5 Hz, 1H), 6.98 (dd, *J* = 5.0, 7.5 Hz, 1H), 5.81-5.76 (m, 2H), 5.37-5.33 (m, 1H), 5.20-5.17 (m, 1H), 4.74-4.68 (m, 1H), 4.61-4.55 (m, 3H), 2.86-2.52 (m, 8H), 2.32-1.78 (m, 19H), 1.68-1.57 (m, 7H), 1.46-1.02 (m, 20H), 0.98-0.82 (m, 17H). LCMS: RT = 7.524 mins, (M+H) = 970.4, Purity = 97%, HPLC Purity = 96%, Chiral HPLC Purity = 95%.

### Example 24: (Hexanoyloxy)methyl (S)-1-(((3-cyanopyridin-2-yl)oxy)methyl)-4-((1R,3aS,5aR,5bR,7aR,11aS,11bR,13aR,13bR)-3a-((2-((1s,4R)-1-hydroxy-4-(methylsulfonyl)cyclohexyl)ethyl)amino)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)cyclohex-3-ene-1-carboxylate

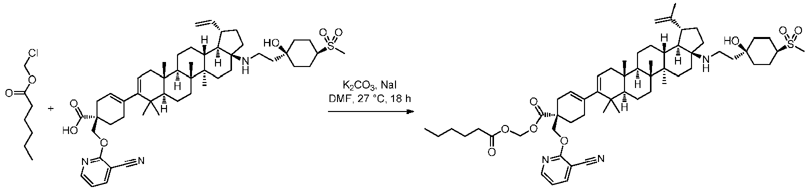

To a stirred solution of (S)-1-(((3-cyanopyridin-2-yl)oxy)methyl)-4-((1R,3aS,5aR,5bR,7aR,11aS,11bR,13aR,13bR)-3a-((2-((1s,4R)-1-hydroxy-4-(methylsulfonyl)cyclohexyl)ethyl)amino)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)cyclohex-3-enecarboxylic acid (150 mg, 0.172 mmol), in DMF (8 mL) were added chloromethyl hexanoate (77 mg, 0.345 mmol), sodium iodide (0.517 mg, 3.45 µmol) and potassium carbonate (119 mg, 0.862 mmol) at 27 °C under nitrogen atmosphere. The reaction mixture was stirred at 27 °C for 18 hr. The progress of the reaction was monitored by TLC (SiO₂, 5% MeOH/DCM, Rf = 0.2, KMnO₄-active & UV-active). On completion, the reaction mixture was poured into ice cold water (150 mL) and extracted with EtOAc (2 × 75 mL). The combined organics were washed with brine (40 mL), dried over Na₂SO₄, filtered and concentrated under reduced pressure to afford the crude residue as pale-yellow gum. The crude compound triturated with chilled n-pentane (3 × 20 mL), filtered and dried under high vacuum to afford (hexanoyloxy)methyl (S)-1-(((3-cyanopyridin-2-yl)oxy)methyl)-4-((1R,3aS,5aR,5bR,7aR,11aS,11bR,13aR,13bR)-3a-((2-((1s,4R)-1-hydroxy-4-(methylsulfonyl)cyclohexyl)ethyl)amino)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)cyclohex-3-ene-1-carboxylate (120 mg, Yield: 65%, off-white solid). ¹H NMR (400 MHz, CDCl₃) δ = 8.32 (dd, *J* = 2.0, 5.0 Hz, 1H), 7.86 (dd, *J* = 2.0, 7.5 Hz, 1H), 6.98 (dd, *J* = 5.0, 7.6 Hz, 1H), 5.81-5.76 (m, 2H), 5.36-5.33 (m, 1H), 5.19-5.17 (m, 1H), 4.74-4.71 (m, 1H), 4.61-4.55 (m, 3H), 2.85-2.65 (m, 6H), 2.57-2.49 (m, 1H), 2.32-1.80 (m, 16H), 1.76-1.70 (m, 1H), 1.65-1.47 (m, 14H), 1.44-1.21 (m, 16H), 1.14-0.82 (m, 22H). LCMS: RT = 7.73 mins, (M+H) = 998.5, Purity = 94%, HPLC Purity = 94%, Chiral HPLC Purity = 97%.

### Example 25: (Isobutyryloxy)methyl (S)-1-(((3-cyanopyridin-2-yl)oxy)methyl)-4-((1R,3aS,5aR,5bR,7aR,11aS,11bR,13aR,13bR)-3a-((2-((1s,4R)-1-hydroxy-4-(methylsulfonyl)cyclohexyl)ethyl)amino)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)cyclohex-3-ene-1-carboxylate

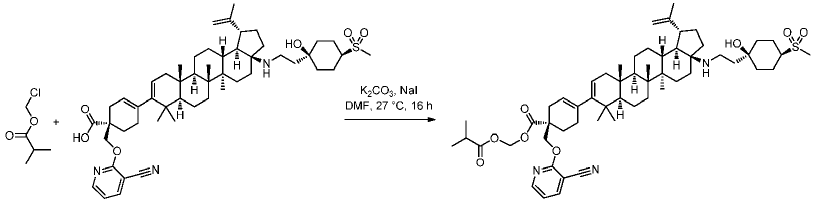

To a stirred solution of (S)-1-(((3-cyanopyridin-2-yl)oxy)methyl)-4-((1R,3aS,5aR,5bR,7aR,11aS,11bR,13aR,13bR)-3a-((2-((1s,4R)-1-hydroxy-4-(methylsulfonyl)cyclohexyl)ethyl)amino)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)cyclohex-3-ene-1-carboxylic acid (200 mg, 0.230 mmol) and chloromethyl isobutyrate (62.8 mg, 0.460 mmol) in DMF (5.0 mL) were added potassium carbonate (159 mg, 1.149 mmol) followed by sodium iodide (6.89 mg, 0.046 mmol) at 0 °C under Nitrogen atmosphere. The reaction mixture was allowed to warm to 27 °C and stirred for 16 hr. The progress of the reaction was monitored by TLC (SiO₂, 5% MeOH/DCM, Rf = 0.4, KMnO₄-active). The reaction mixture was quenched with ice water (50 mL) and extracted with EtOAc (2 × 30 mL). The combined organic layers were washed with brine (50 mL), dried over Na₂SO₄, filtered and concentrated under reduced pressure to give the crude residue as sticky oil. The crude compound was triturated with chilled n-pentane (3 × 10 mL) filtered and dried under high vacuum to afford (isobutyryloxy)methyl (S)-1-(((3-cyanopyridin-2-yl)oxy)methyl)-4-((1R,3aS,5aR,5bR,7aR,11aS,11bR,13aR,13bR)-3a-((2-((1s,4R)-1-hydroxy-4-(methylsulfonyl)cyclohexyl)ethyl)amino)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)cyclohex-3-ene-1-carboxylate (93 mg, Yield: 40%, white solid. ¹H NMR (400 MHz, CDCl₃) δ = 8.32 (dd, *J* = 2.0, 5.0 Hz, 1H), 7.86 (dd, *J* = 2.0, 7.5 Hz, 1H), 6.98 (dd, *J* = 5.0, 7.6 Hz, 1H), 5.81-5.77 (m, 2H), 5.36-5.33 (m, 1H), 5.19-5.17 (m, 1H), 4.74-4.71 (m, 1H), 4.61-4.55 (m, 3H), 2.84-2.64 (m, 7H), 2.57-2.50 (m, 1H), 2.25-1.82 (m, 12H), 1.72-1.38 (m, 22H), 1.32-1.22 (m, 6H), 1.13 (d, *J* = 7.0 Hz, 6H), 1.07-1.01 (m, 6H), 0.98-0.94 (m, 6H), 0.93-0.88 (m, 3H), 0.86-0.82 (m, 3H). LCMS: RT = 7.11 mins, (M+H) = 970.4, Purity = 97%, HPLC Purity = 96%, Chiral HPLC Purity = 94%.

### Example 26: (pivaloyloxy)methyl (R)-4-((1R,3aS,5aR5bR,7aR,11aS,11bR,13aR,13bR)-3a-((2-(1,1-dioxidothiomorpholino)ethyl)amino)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)- I -(fluoromethyl)cyclohex-3-ene- 1-carboxylate.

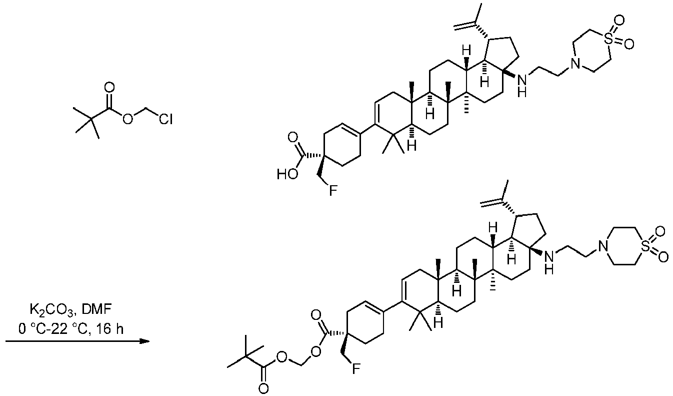

To a stirred solution of (R)-4-((1R,3aS,5aR,5bR,7aR,11aS,11bR,13aR,13bR)-3a-((2-(1,1-dioxidothiomorpholino)ethyl)amino)-5a,5b,8,8,11a-pentamethyl-1-(prop-1-en-2-yl)-2,3,3a,4,5,5a,5b,6, 7,7a,8,11,11a,11b,12,13, 13a,13b-octadecahydro-1H-cyclopenta[a]chrysen-9-yl)-1-(fluoromethyl)cyclohex-3-ene-1-carboxylic acid (500 mg, 0.688 mmol) in N,N-Dimethylformamide (DMF) (5 mL) at 0 °C was added K₂CO₃ (285 mg, 2.063 mmol) followed by chloromethyl pivalate (0.119 mL, 0.825 mmol). The ice bath was removed and the reaction mixture was stirred for 16 h at 22 °C. Progress of the reaction was monitored by LC/MS where product mass was detected by LC/MS. The mixture was diluted with ethyl acetate and washed with water. The ethyl acetate layer was collected, dried with MgSO4, filtered, and the solvent was removed under reduced pressure. The crude product was purified by flash column chromatography on silica gel (0-100% (9:1 DCM:MeOH)/hexanes). The fractions containing product were collected and concentrated under reduced pressure to afford a white powder. The powder was dried in a oven under vacuum for 18 h (50°C) to yield (pivaloyloxy)methyl (R)-4-((1R,3aS,5aR,5bR,7aR,11aS,11bR,13aR,13bR)-3a-((2-(1,1-dioxidothiomorpholino)ethyl)amino)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)-1-(fluoromethyl)cyclohex-3-ene-1-carboxylate (65 mg, 0.072 mmol, 10%). ¹H NMR (500 MHz, DMSO-*d*₆) δ ppm 5.77 - 5.80 (m, 1 H), 5.72 - 5.75 (m, 1 H), 5.26 - 5.30 (m, 1 H), 5.12 - 5.15 (m, 1 H), 4.67 - 4.72 (m, 1 H), 4.41 - 4.58 (m, 3 H), 3.02 - 3.14 (m, 4 H), 2.87 - 2.99 (m, 4 H), 2.55 - 2.66 (m, 3 H), 2.34 - 2.40 (m, 1 H), 1.78 - 2.19 (m, 8 H), 1.28 - 1.77 (m, 15 H), 0.77 - 1.28 (m, 34 H), 0.75 - 0.98 (m, 13 H). LC/MS: RT=2.72 min, (M+H) = 842.45. Purity = 93%.

### Example 27: (heptanoyloxy)methyl (R)-4-((lR,3aS,5aR,5bR,7aR,llaS,llbR,13aR,13bR)-3a-((2-(1,1-dioxidothiomorpholino)ethyl)amino)-5a,5b,8,8,11a-pentamethyl-1-(prop-1-en-2-yl)-2,3,3a,4,5,5a,5b,6,7, 7a,8, 11, 11a,11b, 12, 13, 13 a, 13b-octadecahydro-1H-cyclopenta[a]chrysen-9-yl)-1-(fluoromethyl)cyclohex-3-ene-1-carboxylate.

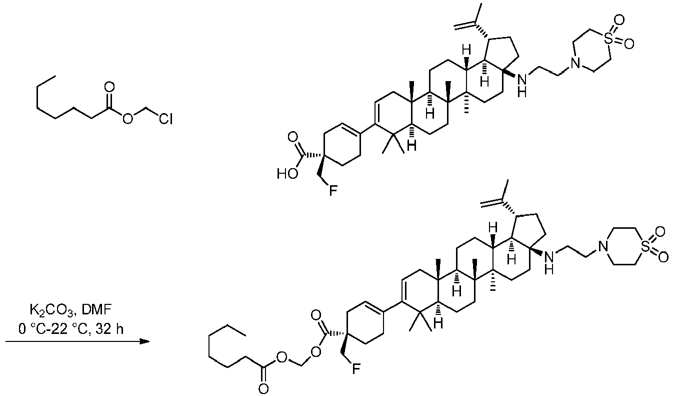

To a stirred solution of (R)-4-((1F,3aS,5aF,5bF,7aR,11aS,11bF,13aF,13bR)-3a-((2-(1,1-dioxidothiomorpholino)ethyl)amino)-5a,5b,8,8,11a-pentamethyl-1-(prop-1-en-2-yl)-2,3,3a,4,5,5a,5b,6, 7,7a,8,11,11a,11b,12,13, 13a,13b-octadecahydro-1H-cyclopenta[a]chrysen-9-yl)-1-(fluoromethyl)cyclohex-3-ene-1-carboxylic acid (258 mg, 0.355 mmol) in N,N-Dimethylformamide (DMF) (5 mL) at 0 °C was added potassium carbonate (147 mg, 1.065 mmol) followed by chloromethyl heptanoate (114 mg, 0.639 mmol). The ice bath was removed and the reaction mixture was stirred for 16 h at 22 °C. Progress of the reaction was monitored by LC/MS where product mass along with starting material was detected. More potassium carbonate (147 mg, 1.065 mmol) followed by chloromethyl heptanoate (114 mg, 0.639 mmol) were added to the reaction mixture and stirred for another 16 hours at 22°C. Mainly product was detected by LC/MS. The mixture was diluted with ethyl acetate:MeOH (9:1) and washed with water. The ethyl acetate layer was collected, dried with MgSO₄, filtered, and the solvent was removed under reduced pressure. The crude product was purified by flash column chromatography on silica gel (0-100% (9:1 DCM:MeOH)/hexanes). The fractions containing product were collected and concentrated under reduced pressure to afford a white powder. The powder was dried in a oven under vacuum for 16 h (50 °C) to yield (heptanoyloxy)methyl (R)-4-((1R,3aS,5aR,5bR,7aR,11aS,11bR,13aR,13bR)-3a-((2-(1,1-dioxidothiomorpholino)ethyl)amino)-5a,5b,8,8,11a-pentamethyl-1-(prop-1-en-2-yl)-2,3,3a,4,5,5a,5b,6,7,7a,8,11, 11a,11b,12, 13, 13a,13b-octadecahydro-1H-cyclopenta[a]chrysen-9-yl)-1-(fluoromethyl)cyclohex-3-ene-1-carboxylate (65 mg, 0.070 mmol, 20%) as a white powder. ¹H NMR (500 MHz, DMSO-*d*₆) δ ppm 5.70 - 5.79 (m, 2 H), 5.25 - 5.30 (m, 1 H), 5.12 (br d, *J*=5.96 Hz, 1 H), 4.67 - 4.72 (m, 1 H), 4.40 - 4.58 (m, 3 H), 3.02 - 3.14 (m, 4 H), 2.86 - 2.99 (m, 4 H), 2.54 - 2.66 (m, 3 H), 2.41 - 2.49 (m, 2 H), 2.28 - 2.39 (m, 3 H), 1.69 - 2.16 (m, 9 H), 0.78 - 1.67 (m, 48 H). LC/MS RT = 3.817 min, (M+) = 869.75. Purity = 94%.

### Example 28: ((isopropoxycarbonyl)oxy)methyl (R)-4-((1R,3aS,5aR,5bR,7aR,11aS,11bR,13aR,13bR)-3a-((2-(1,1-dioxidothiomorpholino)ethyl)amino)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)-1-(fluoromethyl)cyclohex-3-ene-1-carboxylate

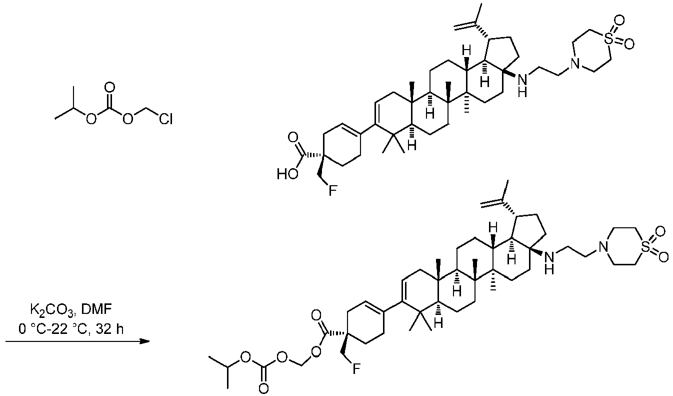

To a stirred solution of (R)-4-((1R,3aS,5aF,5bF,7aR,11aS,11bF,13aF,13bR)-3a-((2-(1,1-dioxidothiomorpholino)ethyl)amino)-5a,5b,8,8,11a-pentamethyl-1-(prop-1-en-2-yl)-2,3,3a,4,5,5a,5b,6, 7,7a,8,11,11a,11b,12,13, 13a,13b-octadecahydro-1H-cyclopenta[a]chrysen-9-yl)-1-(fluoromethyl)cyclohex-3-ene-1-carboxylic acid (350 mg, 0.481 mmol) in N,N-Dimethylformamide (DMF) (5 mL) at 0 °C was added potassium carbonate (200 mg, 1.444 mmol) followed by chloromethyl isopropyl carbonate (77 mg, 0.505 mmol). The ice bath was removed and the reaction mixture was stirred for 18 h at 22 °C. Progress of the reaction was monitored by LC/MS where product mass along with starting material was detected. Potassium carbonate (200 mg, 1.444 mmol) and chloromethyl isopropyl carbonate (77 mg, 0.505 mmol) were added to the reaction mixture and stirred for another 16 h at 22 °C. Mainly product was detected by LC/MS. The mixture was diluted with ethyl acetate and washed with water. The ethyl acetate layer was collected, dried with MgSO₄, filtered, and the solvent was removed under reduced pressure. The crude product was purified by flash column chromatography on silica gel (0-100% (9:1 DCM:MeOH)/hexanes). The fractions containing product were collected and concentrated under reduced pressure to afford a white powder. The powder was dried in a oven under vacuum for 18 h (50 °C) to yield ((isopropoxycarbonyl)oxy)methyl (R)-4-((1R,3aS,5aR,5bF,7aR,11aS,11bF,13aF,13bR)-3a-((2-(1,1-dioxidothiomorpholino)ethyl)amino)-5a,5b,8,8,11a-pentamethyl-1-(prop-1-en-2-yl)-2,3,3a,4,5,5a,5b,6,7,7a,8,11, 11a,11b,12, 13, 13a,13b-octadecahydro-1H-cyclopenta[a]chrysen-9-yl)-1-(fluoromethyl)cyclohex-3-ene-1-carboxylate (60 mg, 0.067 mmol, 14%). ¹H NMR (500 MHz, DMSO-*d*₆) δ ppm 5.74 - 5.78 (m, 1 H), 5.69 - 5.72 (m, 1 H), 5.25 - 5.30 (m, 1 H), 5.10 - 5.16 (m, 1 H), 4.75 - 4.84 (m, 1 H), 4.67 - 4.71 (m, 1 H), 4.40 - 4.60 (m, 3 H), 3.02 - 3.15 (m, 4 H), 2.87 - 2.99 (m, 4 H), 2.55 - 2.67 (m, 3 H), 2.33 - 2.41 (m, 1 H), 1.79 - 2.19 (m, 9 H), 1.70 - 1.77 (m, 1 H), 1.28 - 1.68 (m, 15 H), 0.77 - 1.27 (m, 29 H). LC/MS: RT=2.65 min, (M+H) = 844.45. Purity = 93%.

### Biological data:

### Summary:

A single dose mouse PK study was conducted following an oral dose of the prodrug that was prepared as Example 1, being dosed and measuring parent maturation inhibitor (Ml) #3, and compared to PK parameters with Parent MI#3 being dosed in male CD1 mice. In the current study, the formulation for parent MI #3 was 0.5% Methocel A4M:0.1%Tween 80:99.4% water. For the prodrug of Example 1, the formulation was 2% Dimethylacetamide:10% ethanol: 87.5% PEG 400:0.5% Tween 80.

Following oral administration of the prodrug, all prodrug concentrations at all timepoints were BLQ. As shown in the table below the exposure of the parent MI#3 obtained from dosing the prodrug was higher than that seen with dosing a crystalline suspension of the parent Ml #3.

| **Parameter** | **Unit** | **Parent MI #3 dose d PO** | **Dosed Example 1 PO measured MI #3** |
|---|---|---|---|
| Dose | mg/kg | 5 | 5 |
| Cmax | µg/mL | 1.3 | 2.1 |
| Tmax | h | 4.7 | 4.7 |
| C24 | µg/mL | 0.03 | 0.06 |
| AUC0-last | µg/mL*h | 18 | 29 |
| **AUC0-24h norm** | µg/mL*h/dose | 3.7 | 5.8 |
| **Bioavailability** | %F | 5.0 | 9..0 |

### Biological Data Experiment 2

### Summary

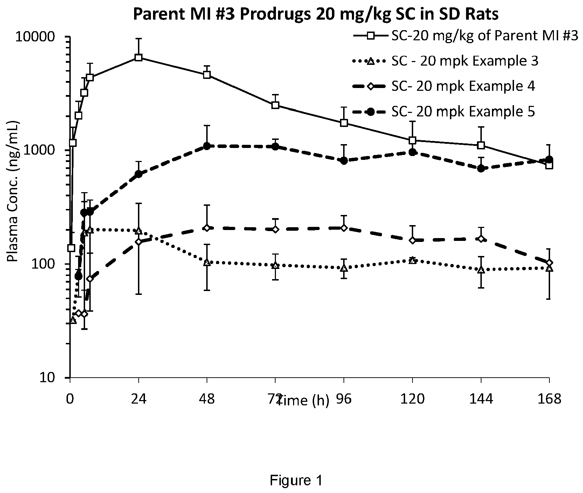

Figure 1 is a graph of concentration vs. time from plasma of MI#3 in ng / mL vs time in hours following subcutaneous (SQ) doses of MI3# or the prodrugs from Examples 3, 4, and 5 as suspensions in 1% Kolliphor P 188/2% PEG 3350/3.5% Mannitol/93.5% water at 20 mg/kg to male CD-1 mice. The data in the figure shows a sustained exposure of Parent MI #3 was observed over 168 hours for all 3 prodrugs. Exposure of Parent MI # 3 decreased 23-fold, 16-fold, and 3-fold after administration of Example 3, 4 and 5, respectively. The presence of the prodrugs was not observed in plasma. Although the exposure is lower than when dosed with parent itself, the half-life of Parent Ml #3 is longer after administration of all three prodrugs showing the potential to be useful for less frequent dosing. The table below shows the PK parameters from the experiments which are summarized in Figure 1.

| **Parameter** | **Unit** | **MI Parent #3 Mouse PK** | | **MI Parent #3 after Example 3 administration** | **MI Parent # 3 after Example 4 administration** | **MI Parent # 3 after Example 5 administration** |
|---|---|---|---|---|---|---|
| Formulation | | Solution: 90%PEG400;10% EtOH | Suspension: 1% Kolliphor P 188/2% PEG 3350/3.5% Mannitol/93.5% Water | Suspension: 1% Kolliphor P 188/2% PEG 3350/3.5% Mannitol/93.5% Water | Suspension: 1% Kolliphor P 188/2% PEG 3350/3.5% Mannitol/93.5% Water | Suspension: 1% Kolliphor P 188/2% PEG 3350/3.5% Mannitol/93.5% Water |
| Dose | mg | **1** | 20 | 20 | 20 | 20 |
| Route | | IV | SQ | SQ | SQ | SQ |
| Cmax | ng/mL | | 6563 | 210 | 267 | 1280 |
| Tmax | h | | 25.167 | 98.3 | 96 | 80 |
| AUClast | ng/mL*h | 64197 | 449442 | 19292 | 27821 | 130603 |
| AUCtot | ng/mL*h | 87808 | 515257 | 30135 | 40081 | 238862 |
| %AUCextra : | | 24.8 | 12.3 | 26.0 | 30.0 | 39.0 |
| t _{(1/2}) | h | 16.3 | 58.3 | 72.0 | 84.0 | 88.0 |
| MRT | h | 22.2 | | | | |
| Clearance | mL/min/kg | 0.20 | | | | |
| Vss | L/kg | 0.25 | | | | |
| Bioavaliability: | %F | | **35** | 7 | 2 | **10** |

### Detailed Protocol:

Subcutaneous (SQ) doses of each of the compounds from Examples 3, 4, and 5 as suspensions (Dose conc. 4 mg / mL, Dose volume 5 mL / kg) in 1% Kolliphor P 188/2% PEG 3350/3.5% Mannitol/93.5% water at 20 mg/kg to 3 male CD-1 mice (Cr1:CD1 (ICR)) Charles River Laboratories, 25-30gs at dose initiation, age as appropriate for weight. The concentration of Parent Ml #3 and prodrugs were measured in the plasma of animals of all three groups at time points of 0.5, 1, 3, 5, 7, 24, 48, 72, 96, 120, 144, and 168 hours. The collection conditions were K₂EDTA and the volume / timepoint was 30 µL. The presence of the prodrugs was not observed in plasma for any group.

### Quantitation by LC-MS/MS

LC-MS/MS methods were developed to quantitate both prodrugs and parent in plasma. The samples were analyzed in duplicate. Two sets of standard curves and three levels of QCs were run in each plate. Both the standards and QCs were run in duplicate. The table below is a summary of the protocols and guidance that was supplied to the scientists who carried out the experiments.

| | | |
|---|---|---|
| **Dose Administration** | | • The dose formulation will be administered as a single subcutaneous injection. |
| | | • The volume of each dose delivered will be based on each individual animal's body weight recorded prior to dose administration. |
| **Clinical Observations** | | • Observations were recorded after dosing and at each sample collection time point. Any abnormalities or the absence of abnormalities will be reported. Abnormalities will be reported to the study director. |
| **Body Weights** | | • Body weights will be recorded before dosing |
| **Blood collection and processing** | | • Blood samples will be collected by tail snip or from a submandibular vein. |
| | | • 30 µL of whole blood will be used at each time point using a capillary collection tube (Sarstedt) containing K₂EDTA anticoagulant. |
| | | • The whole blood samples will be centrifuged at 2200 x g for 10 minutes at 5 ± 3°C to isolate plasma. |
| | | • The resulting plasma will be transferred to individual polypropylene tubes in a 96 well plate format and frozen on dry ice until storage nominally - 80°C prior to analysis. |
| **Reporting** | A signed life summary report will be provided following completion of all in-life procedures. The summary report will include a description of dose formulation, in-life dosing procedures, and sample collections accompanied by tabular data detailing dose administration, sample collection times, any abnormal clinical observations, and sample shipping details. | |

### Biological Data Experiment 3

### Summary:

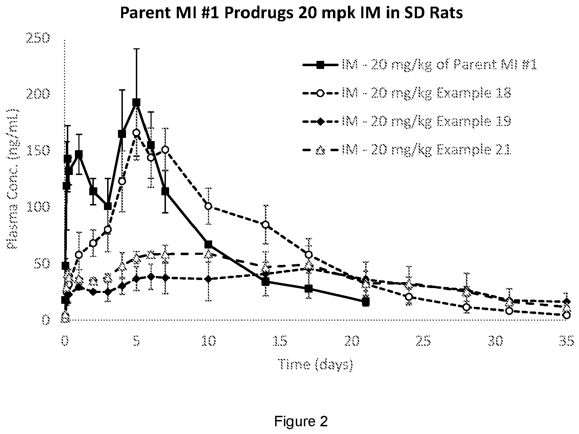

Figure 2 is a concentration vs time curve of the plasma concentrations of parent Ml #1 following a single Intramuscular (IM) administration of the Prodrugs and parent Ml #1 to male SD rats. The results of the study examined at day 35 post dose showed that several of the prodrugs showed a steady exposure with a longer half-life and relatively low peak to trough ratio indicating they could have utility for use after an IM injection and could potentially be dosed less frequently than parent MI #1 or most currently approved HIV drugs. Additional pharmacokinetic data collected from the same experiments summarized in Figure 2 is contained in the table below.

| **Example #** | **18** | **19** | **21** |
|---|---|---|---|
| T_{max(day)} | 4.67 | 17 | 10 |
| C_{max(ng/mL)} | 171 | 45.9 | 59 |
| AUC_{INF (day)} | 2100 | 1393 | 1682 |
| AUC_{INF (h)} | 50400 | 33432 | 40374 |
| %F | 93.7 | 62.1 | 75.0 |

### Detailed protocol:

Animal strain: Sprague Dawley (SD) rat, purchased from Shanghai SLAC Laboratory Animal Co., Ltd. Sex: male; Age: 6-8 weeks; BW: 200-250 g, N= 30 rats (N= 3 per prodrug); Food status: Free access to food and water for the entire in-life phase.
Dose: 20 mg/kg (2 mL/kg, 10 mg/mL), IM injection; Formulation: 1%Kolliphor P188+4.5%Mannitol+94.5%Milli-Q Water;
Sampling Time Point:
   N=3 rats/time point for each compound via IM administration
Sampling at 0.5, 1, 3, 5 and 7 hr, 1, 2, 3, 4, 5, 6 and 7 days post dose then 2x/week for a total of 8 weeks, 12*8 time points, serial bleeding for plasma only.
* Weekly interim analysis of the plasma samples (12 time points) to get an early read on the PK of the construct
Blood collection and processing: The animal was restrained manually and approximately 150 µL blood per time point was collected in tubes containing K2EDTA anticoagulant. The sample was centrifuged at 2200 g for 10 min at 2-8°C for plasma. The plasma samples were stored immediately at -70°C
Injection site tissue collection: At the end of the study, injection site was collected and stored at -70°C until analysis for prodrug or parent concentrations.
Clinical observation: Animals were monitored twice daily, and any clinical observations were recorded and shared. Injection site was observed for any apparent changes.
Cageside observations: Cageside observations were made for each animal once daily, see below for details. Injection site was observed for any apparent changes. Abnormal findings were recorded as they were observed for mortality, abnormalities, and signs of pain or distress.
   I. Mild:
      Pay closer attention and increase monitoring frequency;
   II. Moderate to Severe:
      Consult with DVM for euthanasia advice before animals are sacrificed.

Unscheduled Euthanasias and Deaths: A necropsy was conducted on all animals that died or were euthanized (via CO2) due to poor condition at an unscheduled interval. Macroscopic observations were made and lesions and major organs (lung, liver, kidney, heart, spleen), saved in formalin. The time interval from found-dead/euthanasia to necropsy was recorded. If necropsy is delayed and longest duration of any foreseen delay to necropsy, storage condition of the animals will be recorded. In addition, a plasma sample for parent/prodrug exposure was obtained from those animals that were euthanized due to poor condition.

### Quantitation by LC-MS/MS

LC-MS/MS method was developed to quantitate both prodrugs and parent in plasma. The samples were analyzed in duplicate. Two sets of standard curves and three levels of QCs were run in each plate. Both the standards and QCs were run in duplicate.

### Pharmacokinetics Analysis

WinNonlin V 6.4 statistics software (Pharsight Corporation, California, USA) was used to generate pharmacokinetics parameters (Tmax, Cmax, T1/2, AUC, MRT etc).

### Data Presentation

Pharmacokinetic data and in-life observations in PK summary were collected and compiled in an Excel file. This data is summarized in the Figures, tables and graphs in this application.

Instructions provided in advance for any clinical observations that might have arisen in Study are in the following table. SD stands for study director.

| **Items** | | **Description** | **Notice** | **To-do list** |
|---|---|---|---|---|
| 1 | Weight | Weight loss 0%~20% | Compare daily weight with the 1st day; Weigh the animal at same time each day | Inform SD once weight loss occurs |
| | | Weight loss >20% | | Communicate with SD. Euthanasia recommended |
| 2 | Activity | Mild reduction: relatively reduced activities compared with normal animals | | Inform SD |
| | | Medium reduction: lie still at the bottom of the cage, move with outside touch | Inform SD timely once medium or severe symptoms last >0.5 hr; increase observation frequency and keep communicating with SD | Inform SD |
| | | Severe reduction: lie still at the bottom of the cage, no reaction to outside touch | | Inform SD, euthanasia recommended once symptoms last >1 hr |
| | | Increase: significant moving distance increases in unit time; Jump up and down | Increase observation frequency once symptoms last >10 min | Inform SD, euthanasia recommended once symptoms last >1 hr |
| 3 | Righting reflex | Lack of righting reflex: animal cannot stand with its paw once turned over | | Inform SD, euthanasia recommended once symptoms last >1 hr |
| 4 | Circling | circling clockwise or anti-clockwise in the cage | | Inform SD |
| 5 | Hunched posture | Mild: tentatively hunched post dosing | Hunched posture with messed fur | Inform SD |
| | | Medium: paroxysmal hunched posture | | Inform SD |
| | | Severe: continuous hunched posture | | Communicate with SD, euthanasia recommended once symptoms>4 hr |
| 6 | Fur/hair | Mild erection: partial erection of back fur | Fur erected from the root. Do not confused with dirty fur; usually correlated with diseases and/or pain | Inform SD |
| | | Medium erection: mostly erection of back fur | | Inform SD |
| | | Severe erection: mostly erection of back fur with dehydration (skin shrinkage), usually accompanied with hunched posture | | Communicate with SD, euthanasia recommended |
| 7 | Respiration | Increased respiration rate: shallow breathing with high frequency | Compared with normal animals, judged by abdominal undulation frequency | Inform SD |
| | | Dyspnoea: difficult inhalation-gasp | | Communicate with SD, euthanasia recommended once symptoms>1 hr |
| 8 | Limbs | Lie down on one side: animal cannot stand with paws | | Inform SD, euthanasia recommended |
| | | | | once symptoms>1 hr |
| | | Rigid limbs: stiff and rigid limbs | | Inform SD, euthanasia recommended once symptoms>15 min |
| 9 | Convulsion | Paroxysmal convulsion | | Inform SD |
| | | continuous convulsion | | Inform SD, euthanasia recommended once symptoms>10 min |
| 10 | Feces | Soft feces: shaped, softer than normal | | Inform SD |
| | | Loose feces: unshaped, pasty | | Inform SD |
| | | Water feces: unshaped, water-like, bloody | | Inform SD |
| 11 | Urine | Color: light red, reddish brown, light green, blackish green, etc. | Color judgment | Inform SD |
| 12 | Nose discharge | Discharge or secretion outflows from the nose (noticed color) | Observation of concomitant clinical symptoms | Inform SD |
| 13 | Eye discharge | Discharge or other stuffs in the eyes | Observation of concomitant clinical symptoms | Inform SD |
| 14 | Mouth Discharge | Stained discharge or stuff in the mouth | | Inform SD |
| | | Excessive salivation: saliva secretion increased | Keep observation to see if continuously outflow with large amount of liquid | Inform SD, euthanasia is recommended once >1 hr |
| 15 | Anal discharge | Liquid discharge, mucus discharge (notice color) | | Inform SD |
| 16 | Warped tail (Tubular tail) | Intermittent | Record duration | Inform SD |
| | | Continuous | Record duration | Inform SD |
| 17 | Temperature | Cold touch: touch of the animal is colder than normal | | Inform SD |

## Claims

1. A compound of Formula I or a pharmaceutically acceptable salt thereof 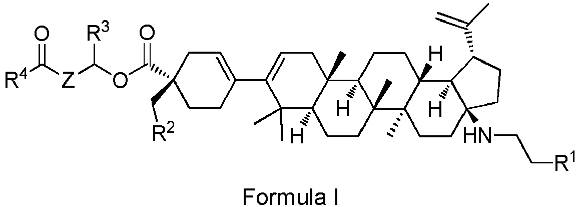
wherein R¹ is 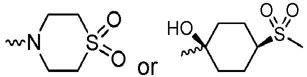 where the squiggly line indicates the point of attachment to the rest of the molecule;
R² is F or 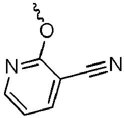 where the squiggly line indicates the point of attachment to the rest of the molecule;
R³ is H or CH₃;
Z is O; and
R⁴ is C₁₋₃₀alkyl.

2. A compound or pharmaceutically acceptable salt thereof as defined in claim 1 for use in therapy.

3. A pharmaceutical composition comprising a compound or a pharmaceutically acceptable salt thereof according to claim 1.

4. A pharmaceutical composition according to claim 3 wherein said composition is in the form of a tablet, a capsule, or a form suitable for injection.

5. A compound or pharmaceutically acceptable salt thereof as defined in claim 1 for use in treating an HIV infection in a human.

6. A compound or pharmaceutically acceptable salt for use according to claim 5 further comprising administering to the subject at least one other agent used for treatment of AIDS or HIV infection selected from the group consisting of nucleoside HIV reverse transcriptase inhibitors, non-nucleoside HIV reverse transcriptase inhibitors, HIV protease inhibitors, HIV fusion inhibitors, HIV attachment inhibitors, CCR5 inhibitors, CXCR4 inhibitors, HIV budding or maturation inhibitors, and HIV integrase inhibitors.

7. A compound or pharmaceutically acceptable salt thereof for use according to claim 6 wherein said additional agent is dolutegravir or cabotegravir.

## Patentansprüche

1. Verbindung der Formel I oder ein pharmazeutisch akzeptables Salz davon 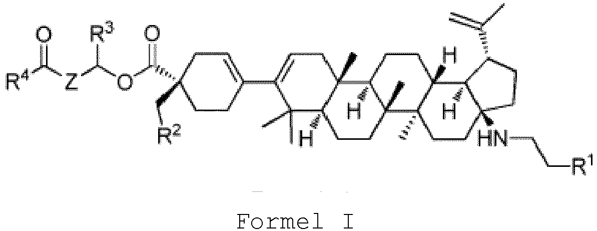
worin R¹ 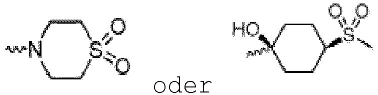 ist, wobei die geschlängelte Linie den Punkt der Bindung an den Rest des Moleküls anzeigt;
R² ist F oder 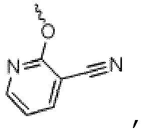 wobei die geschlängelte Linie den Punkt der Bindung an den Rest des Moleküls anzeigt;
R³ ist H oder CH₃;
Z ist O; und
R⁴ ist C₁₋₃₀-Alkyl.

2. Verbindung oder pharmazeutisch akzeptables Salz gemäß Anspruch 1 zur Verwendung in der Therapie.

3. Pharmazeutische Zusammensetzung umfassend eine Verbindung oder ein pharmazeutisch akzeptables Salz davon gemäß Anspruch 1.

4. Pharmazeutische Zusammensetzung gemäß Anspruch 3, wobei die Zusammensetzung in Form einer Tablette, einer Kapsel, oder einer zur Injektion geeigneten Form ist.

5. Verbindung oder pharmazeutisch akzeptables Salz davon gemäß Anspruch 1 zur Verwendung bei der Behandlung einer HIV-Infektion bei einem Menschen.

6. Verbindung oder pharmazeutisch akzeptables Salz zur Verwendung gemäß Anspruch 5, ferner umfassend die Verabreichung mindestens eines weiteren Mittels, das zur Behandlung von AIDS oder einer HIV-Infektion verwendet wird, ausgewählt aus der Gruppe, bestehend aus Nukleosid-HIV-Reverse-Transkriptase-Inhibitoren, Nicht-Nukleosid-HIV-Reverse-Transkriptase-Inhibitoren, HIV-Protease-Inhibitoren, HIV-Fusionsinhibitoren, HIV-Anheftungsinhibitoren, CCRS-Inhibitoren, CXCR4-Inhibitoren, HIV-Knospungs- oder Reifungsinhibitoren und HIV-Integrase-Inhibitoren, an den Patienten.

7. Verbindung oder pharmazeutisch akzeptables Salz davon zur Verwendung gemäß Anspruch 6, wobei das weitere Mittel Dolutegravir oder Cabotegravir ist.

## Revendications

1. Composé de Formule 1 ou un sel pharmaceutiquement acceptable de celui-ci 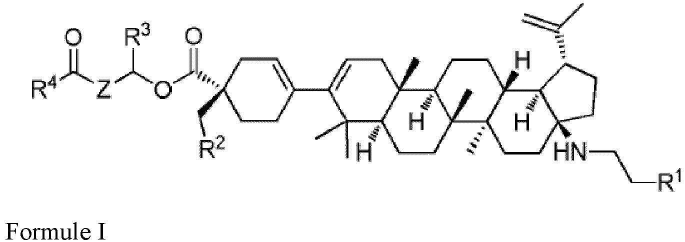
dans lequel R¹ est 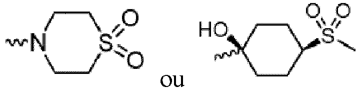 où la ligne sinueuse indique le point de fixation au reste de la molécule ;
R² est F ou 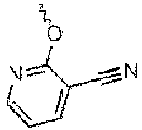 où la ligne sinueuse indique le point de fixation au reste de la molécule ;
R³ est H ou CH₃ ;
Z est O ; et
R⁴ est un alkyle en C₁₋₃₀.

2. Composé ou sel pharmaceutiquement acceptable de celui-ci selon la revendication 1 pour une utilisation en thérapie.

3. Composition pharmaceutique comprenant un composé ou un sel pharmaceutiquement acceptable de celui-ci selon la revendication 1.

4. Composition pharmaceutique selon la revendication 3 dans laquelle ladite composition est sous la forme d'un comprimé, d'une capsule, ou sous une forme appropriée pour une injection.

5. Composé ou sel pharmaceutiquement acceptable de celui-ci selon la revendication 1 pour une utilisation dans le traitement d'une infection par le VIH chez un être humain.

6. Composé ou sel pharmaceutiquement acceptable pour une utilisation selon la revendication 5 comprenant en outre l'administration au sujet d'au moins un autre agent utilisé pour le traitement du SIDA ou d'une infection par le VIH choisi dans le groupe constitué des inhibiteurs nucléosidiques de la transcriptase inverse du VIH, des inhibiteurs non nucléosidiques de la transcriptase inverse du VIH, des inhibiteurs de la protéase du VIH, des inhibiteurs de fusion du VIH, des inhibiteurs de fixation du VIH, des inhibiteurs de CCR5, des inhibiteurs de CXCR4, des inhibiteurs de bourgeonnement ou de maturation du VIH, et des inhibiteurs de l'intégrase du VIH.

7. Composé ou sel pharmaceutiquement acceptable de celui-ci pour une utilisation selon la revendication 6 dans lequel ledit agent additionnel est le dolutégravir ou le cabotégravir.
